(19) Europäisches Patentamt
European Patent Office
Office européen des brevets

(11) Veröffentlichungsnummer: **0 117 403**
**B1**

(12) # EUROPÄISCHE PATENTSCHRIFT

(45) Veröffentlichungstag der Patentschrift: **01.06.88**

(21) Anmeldenummer: **84100381.7**

(22) Anmeldetag: **16.01.84**

(51) Int. Cl.⁴: **C 07 D 237/04**, C 07 D 237/14, A 61 K 31/50, C 07 D 237/26, C 07 D 401/12, C 07 D 401/14, C 07 D 403/12, C 07 D 405/12, C 07 D 413/12

(54) **6-(Acylaminoaryl)-3(2H)-pyridazinonderivate, ihre Herstellung und Verwendung.**

(30) Priorität: **22.01.83 DE 3302021**

(43) Veröffentlichungstag der Anmeldung: **05.09.84 Patentblatt 84/36**

(45) Bekanntmachung des Hinweises auf die Patenterteilung: **01.06.88 Patentblatt 88/22**

(84) Benannte Vertragsstaaten: **AT BE CH DE FR GB IT LI NL SE**

(56) Entgegenhaltungen:
**EP - A - 0 107 735**
**DE - A - 2 123 246**
**DE - A - 2 304 977**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Rossy, Phillip A., Dr., 39, Forest Drive, Hillsdale, N.J. 07642 (US)**
Erfinder: **Thyes, Marco, Dr., Thorwaldsenstrasse 1, D-6700 Ludwigshafen (DE)**
Erfinder: **Franke, Albrecht, Dr., Mandelring 11, D-6706 Wachenheim (DE)**
Erfinder: **Koenig, Horst, Prof.Dr., Pariser Strasse 4, D-6700 Ludwigshafen (DE)**
Erfinder: **Lehmann, Hans Dieter, Prof.Dr., Im Hefen 15, D-6945 Hirschberg (DE)**
Erfinder: **Gries, Josef, Dr., Roemerweg 43, D-6706 Wachenheim (DE)**
Erfinder: **Friedrich, Ludwig, Dr., Nibelungenstrasse 8a, D-6831 Bruehl (DE)**
Erfinder: **Lenke, Dieter, Prof.Dr., Kekuleplatz 1, D-6700 Ludwigshafen (DE)**

EP 0 117 403 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue 6-(Acylaminoaryl)-3(2H)-pyridazinonderivate, Verfahren zu deren Herstellung und deren Verwendung bei der Bekämpfung von Krankheiten.

Pharmakologisch aktive 6-(Acylaminoaryl)-4,5-dihydro-3(2H)-pyridazinone sind bereits bekannt, vgl. DE-OS 1 670 158, DE-OS 2 123 246, DE-OS 2 150 436, DE-OS 2 157 453, DE-OS 2 304 977, DE-OS 2 727 481, DE-OS 2 854 191, DE-OS 2 854 475, DE-OS 3 022 176, DE-OS 3 022 177, DE-OS 3 033 702, DE-OS 3 209 158, DE-OS 3 209 159, JP-OS 53.124-279, JP-OS 58 008 015, JP-OS 58 008 016 sowie die nicht vorveröffentlichten Schriften WP 83 01 447 und US-PS 4 397 854. Ebenso sind auch bereits 6-(Acylaminoaryl)-3(2H)-pyridazinone mit pharmakologischer Aktivität bekannt, vgl. JP-OS 58 008 015, JP-OS 58 008 016, US 4 397 854, Journal of Medicinal Chemistry, Band 17 (1974), S. 273–281.

Die in diesen Publikationen beschriebenen Substanzen besitzen blutdrucksenkende, entzündungshemmende, membranstabilisierende, thrombozytenaggregationshemmende, cardiotone und/oder coronarerweiternde Eigenschaften oder wirken cardiovasculär bzw. antiallergisch.

Es wurden nun gefunden, dass 6-(Acylaminoaryl)-3(2H)-pyridazinonderivate der Formel I

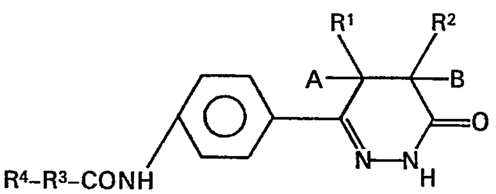

(I),

in der

A und B Wasserstoffatome sind oder gemeinsam eine Bindung bilden,

$R^1$ ein Wasserstoffatom oder eine $C_1$–$C_3$-Alkylgruppe darstellt,

$R^2$ ein Wasserstoffatom ist oder – falls A und B Wasserstoffatome sind – $R^1$ und $R^2$ zusammen einen $C_{1-4}$-Alkylenrest bedeuten,

$R^3$ eine geradkettige $C_{1-4}$-Alkylengruppe, die durch 1 bis 2 $C_{1-5}$-Alkylgruppen substituiert sein kann, darstellt und

$R^4$ a) für einen Pyrrol-1-yl-, Imidazol-1-yl-, Pyrazol-1-yl- oder 1,2,4-Triazol-1-yl-Rest steht, oder

b) eine Gruppe der Formel II

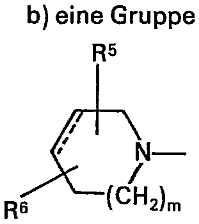

(II)

darstellt, worin

die gestrichelte Linie eine zusätzliche Bindung bedeuten kann, $R^5$ und $R^6$ gleich oder verschieden sind und Wasserstoffatome, $C_{1-4}$-Kohlenwasserstoffreste, die gegebenenfalls durch eine $C_{3-8}$-Cycloalkylgruppe oder einen Phenylrest, der gegebenenfalls 1 bis 3 $C_{1-4}$-Alkyl-, $C_{1-4}$-Alkoxy-, Trifluormethyl- und/oder Nitrogruppen und/oder Halogenatome aufweist, substituiert sind, $C_{3-8}$-Cycloalkyl-, Hydroxy-, Trifluormethyl-, $C_{1-4}$-Acyl-, Carboxy-, ($C_{1-5}$-Alkoxy)carbonyl, Cyangruppen oder gegebenenfalls 1-bis 3fach durch Halogenatome oder $C_{1-4}$-Alkyl, $C_{1-4}$-Alkoxy-, Hydroxy-, Trifluormethyl-, $C_{1-4}$-Acyl-, Carboxy-, ($C_{1-5}$-Alkoxy)-carbonyl-, Cyan-, Nitro-, Amino-, $C_{1-4}$-Alkylamino, Di-$C_{1-4}$-alkylamino, Anilino- oder $C_{1-4}$-Acylaminogruppen substituierte $C_6$-$C_{10}$-Aryl- oder 5-bis 6gliedrige gegebenenfalls benzokondensierte Heteroarylreste mit 1 bis 3 Heteroatomen bedeuten, oder

Gruppen der Formel $R^7R^8N$- darstellen, in der $R^7$ und $R^8$ gleich oder verschieden sind und Wasserstoffatome, $C_{1-8}$-Alkylreste, die gegebenenfalls durch einen Phenylrest, der gegebenenfalls 1 bis 3 $C_{1-4}$-Alkyl-, $C_{1-4}$-Alkoxy-, Trifluormethyl- und/oder Nitrogruppen und/oder Halogenatome aufweist, substituiert sind, Phenylgruppen, die durch 1 bis 3 Halogenatome, $C_{1-4}$-Alkyl, $C_{1-4}$-Alkoxy-, Trifluormethyl-, $C_{1-4}$-Acyl-, Carboxy-, ($C_{1-5}$-Alkoxy)-carbonyl-, Cyan- und/oder Nitrogruppen substituiert sein können, oder $C_{1-8}$-Acyl- oder $C_{6-10}$-Aroylreste bedeuten, wobei die Aroylgruppe 1- bis 3fach durch Halogenatome, $C_{1-4}$-Alkyl-, $C_{1-4}$-Alkoxy-, Hydroxy-, Trifluormethyl-, $C_{1-4}$-Alkylthio-, $C_{1-4}$-Alkylsulfoxy-, $C_{1-4}$-Alkylsulfonyl-, $C_{1-4}$-Acyl-, Carboxy-, ($C_{1-5}$-Alkoxy)-carbonyl-, Cyan-, Nitro-, Amino-, $C_{1-4}$-Alkylamino-, Di-$C_{1-4}$-Alkylamino- oder $C_{1-4}$-Acylaminogruppen substituiert sein kann, oder die Gruppe $R^7R^8N$- für einen Benzimidazol-2-on-1-yl-rest steht, oder

$R^5$ und $R^6$ zusammen eine $C_{1-4}$-Alkylenkette bilden, wobei der Bicyclus gegebenenfalls durch 1 bis 3 $C_{1-3}$-Alkylgruppen substituiert ist, und

m = 0, 1, 2 oder 3 ist, oder

c) eine Gruppe der Formel III

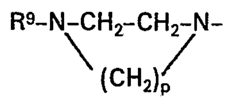

(III)

darstellt, in der $R^9$ einen $C_{1-14}$-Kohlenwasserstoffrest, der gegebenenfalls durch eine Hydroxygruppe, eine $C_{3-8}$-Cycloalkylgruppe, wobei eine $C_{5-8}$-Cycloalkylgruppe benzokondensiert sein kann, oder eine $C_{6-10}$-Arylgruppe, die 1 bis 3 $C_{1-4}$-Alkyl-, $C_{1-4}$-Alkoxy-, Trifluormethyl-, Nitro-, Hydroxy-, $C_{1-4}$-Acyl-, Carboxy-, ($C_{1-5}$-Alkoxy)-carbonyl-, Amido-, N-($C_{1-4}$-Alkyl)amido-, N,N-Di-($C_{1-4}$-Alkyl)-amido-, Tri($C_{1-4}$-Alkyl)-silyl-, Cyan-, Amino-, $C_{1-4}$-Alkylamino-, Di-($C_{1-4}$-alkyl)amino-, $C_{1-4}$-Acylaminogruppen und/oder Halogenatome enthalten kann, substituiert ist;

eine $C_6$-$C_{10}$-Aroylgruppe, die gegebenenfalls durch 1 bis 3 $C_{1-4}$-Alkyl-, $C_{1-4}$-Alkoxy-, Trifluormethyl-, Nitro-, Hydroxy-, $C_{1-4}$-Acyl-, Carboxy-, $C_{1-5}$-Alkoxycarbonyl-, N,N-Di-($C_{1-4}$-alkyl)amido-, Cyan-, Di-($C_{1-4}$-alkyl)aminogruppen und/oder Halogenatome substituiert ist;

eine Heteroaroylgruppe mit 5 bis 6 Ringgliedern, die 1 bis 3 Heteroatome enthalten kann und gegebenenfalls benzokondensiert ist; eine $C_{3-12}$-Cy-

cloalkyl-, $C_{1-8}$-Acyl-, ($C_{1-5}$-Alkoxy)-carbonylgruppe oder eine gegebenenfalls 1- bis 3fach durch Halogenatome oder $C_{1-4}$-Alkyl-, $C_{1-4}$-Alkoxy-, Hydroxy-, Trifluormethyl-, $C_{1-4}$-Acyl-, Carboxy-, ($C_{1-5}$-Alkoxy)-carbonyl-, Cyan-, Nitro-, Amino-, $C_{1-4}$-Alkylamino-, Di-$C_{1-4}$-alkylamino-, Arylamino- oder $C_{1-4}$-Acylaminogruppen substituierten $C_{6-10}$-Aryl- oder einen 5-bis 6gliedrigen, gegebenenfalls benzokondensierten Heteroarylrest, der 1 bis 3 Stickstoffatome und gegebenenfalls ein Sauerstoffatom oder ein Schwefelatom enthält, bedeutet, und

p 2 oder 3 ist, oder

d) eine Gruppe der Formel IV

$-NR^{10}R^{11}$ (IV),

in der $R^{10}$ und $R^{11}$ Wasserstoffatome, $C_{1-12}$-Kohlenwasserstoffreste, die gegebenenfalls durch einen oder zwei Phenylreste, die gegebenenfalls 1 bis 3 $C_{1-4}$-Alkyl-, $C_{1-4}$-Alkoxy-, Trifluormethyl- und/oder Nitrogruppen und/oder Halogenatome aufweisen,

eine Naphthylgruppe, eine $C_{3-8}$-Cycloalkylgruppe, die 1 bis 3 $C_{1-4}$-Alkylgruppen enhalten kann, wobei $C_{5-8}$-Cycloalkylgruppen benzokondensiert sein können, oder eine $C_{7-10}$-Bicycloalkylgruppe, die 1 bis 3 $C_{1-4}$-Alkylgruppen enthalten kann, substituiert sind;

$C_{3-12}$-Cycloalkylgruppen, die durch 1 bis 3 $C_{1-4}$-Kohlenwasserstoffreste, die eine Phenylgruppe, die 1- bis 3fach durch $C_{1-4}$-Alkyl-, $C_{1-4}$-Alkoxy-, Trifluormethyl-, Nitril-, Nitrogruppen und/oder Halogenatome substituiert sein kann, oder eine $C_{3-6}$-Cycloalkylgruppe enthalten können, oder durch eine Phenylgruppe, die 1 bis 3 $C_{1-4}$-Alkyl-, $C_{1-4}$-Alkoxy-, Trifluormethyl-, Nitril-, Nitrogruppen und/oder Halogenatome enthalten kann, oder eine $C_{3-8}$-Cycloalkylgruppe substituiert sein können;

$C_{7-10}$ bi- oder tricyclische Alkylreste, die durch 1 bis 3 $C_{1-4}$-Alkylgruppen substituiert sein können, oder

benzokondensierte $C_{5-7}$-Cycloalkylgruppen bedeuten, oder

e) eine Gruppe der Formel V

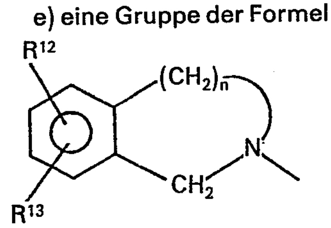

(V)

darstellt, in der $R^{12}$ und $R^{13}$ Wasserstoffatome, Halogenatome, $C_{1-4}$-Alkyl- oder $C_{1-4}$-Alkoxyreste bedeuten, und n für 1, 2 oder 3 steht, oder

f) eine Gruppe der Formel VI

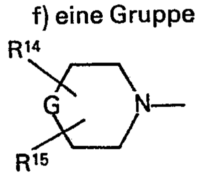

(VI)

in der G ein Sauerstoff- oder Schwefelatom und $R^{14}$ und $R^{15}$ Wasserstoffatome oder $C_{1-4}$-Alkylgruppen bedeuten, darstellt, mit der Massgabe, dass,

wenn $R^1$, $R^2$, A und B Wasserstoffatome bedeuten, $R^3$ keine gegebenenfalls durch eine $C_{1-5}$-Alkylgruppe substituierte Methylgruppe bedeuten soll und

wenn $R_2$, A und B Wasserstoffatome bedeuten, $R^1$ ein Wasserstoffatom oder eine $C_{1-3}$-Alkylgruppe bedeutet und $R^3$ eine gegebenenfalls durch einen $C_{1-4}$-Alkylrest substituierte Methylengruppe bedeutet, $R^4$ nicht eine Gruppe der Formel IV darstellen soll, in der $R^{10}$ und $R^{11}$ Wasserstoffatome oder unsubstituierte $C_{1-4}$-Alkylgruppen bedeuten, sowie deren Salze mit physiologisch verträglichen Säuren wertvolle pharmakologische Eigenschaften aufweisen.

$R^1$ bedeutet vorzugsweise ein Wasserstoffatom, eine Methylgruppe oder gemeinsam mit $R^2$ einen $C_{1-2}$-Alkylenrest. Besonders bevorzugte Bedeutungen für $R^1$ sind: ein Wasserstoffatom oder, falls A und B Wasserstoffatome bedeuten, eine Methylgruppe.

Bedeuten $R^1$ und $R^2$ zusammen eine $C_{1-4}$-Alkylengruppe, so ist ganz besonders die Methylengruppe zu nennen.

A und B bedeuten vorzugsweise Wasserstoffatome oder eine gemeinsame Bindung, besonders bevorzugt stellen sie jedoch Wasserstoffatome dar.

$R^3$ bedeutet bevorzugt eine geradkettige $C_{1-4}$-Alkylengruppe, die durch eine $C_{1-3}$-Alkylgruppe oder zwei Methylgruppen substituiert sein kann. Besonders bevorzugt steht $R^3$ für eine $C_{1-3}$-Alkylengruppe, insbesondere die Methylen- oder Ethylengruppe, die durch eine Methylgruppe substituiert sein kann.

Von den für $R^4$ unter a) genannten Resten ist der 1-Imidazol-1-ylrest bevorzugt.

Bedeutet $R^4$ eine Gruppe der Formel II, so steht $R^5$ bevorzugt für ein Wasserstoffatom, eine $C_{1-4}$-Alkylgruppe, die gegebenenfalls durch einen Phenylrest substituiert ist, eine $C_{3-6}$-Cycloalkylgruppe, eine Phenylgruppe, die gegebenenfalls durch 1 bis 2 Halogenatome, $C_{1-4}$-Alkyl-, $C_{1-4}$-Alkoxygruppen und/oder eine Trifluormethyl-, Nitril- und/oder Nitrogruppe substituiert sein kann, oder eine Gruppe der Formel $R^7R^8N$-, in der $R^7$ ein Wasserstoffatom oder eine Phenylgruppe, die 1- bis 2fach durch Halogenatome, $C_{1-4}$-Alkyl-, $C_{1-4}$-Alkoxygruppen und/oder eine Nitril- und/oder Nitrogruppe substituiert sein kann, und $R^8$ eine $C_{1-4}$-Acyl- oder Benzoylgruppe bedeutet oder $R^7$ und $R^8$ zusammen mit dem Stickstoffatom eine Benzimidazol-2-on-1-ylgruppe bilden.

$R^6$ bedeutet vorzugsweise ein Wasserstoffatom, eine $C_{1-4}$-Alkyl-, eine Hydroxy-, eine $C_{1-4}$-Acyl-, eine ($C_{1-5}$-Alkoxy)-carbonyl- oder eine Cyanogruppe.

Besonders bevorzugte Reste für $R^5$ sind eine durch Phenyl substituierte $C_{1-4}$-Alkylgruppe, eine gegebenenfalls durch ein Halogenatom substituierte Phenylgruppe oder eine Gruppe der Formel $R^7R^8N$, in der $R^7$ ein Phenylgruppe und $R^8$ eine

$C_{1-4}$-Acylgruppe bedeutet. Von diesen Resten sind die Benzylgruppe und der Phenylrest, der gegebenenfalls durch ein Halogenatom substituiert ist, besonders erwähnenswert.

Bilden $R^5$ und $R^6$ gemeinsam eine $C_{1-4}$-Alkylenkette, sind unsubstituierte Ketten oder solche bevorzugt, die durch 1 bis 3 Methylgruppen substituiert sind.

m bedeutet vorzugsweise 0,1 oder 2, besonders bevorzugt 1.

Bedeutet $R^4$ eine Gruppe der Formel III, so sind folgende Reste für $R^9$ bevorzugt: Ein $C_{1-3}$-Kohlenwasserstoffrest, der durch einen Naphthylrest oder einen Phenylrest substituiert ist, der gegebenenfalls 1-bis 2fach durch Halogenatome, $C_{1-4}$-Alkyl-, $C_{1-4}$-Alkoxygruppen und/oder durch eine Trifluormethyl-, Hydroxy-, $C_{1-4}$-Acyl-, ($C_{1-5}$-Alkoxy)-carbonyl-, Nitro- und/oder Nitrilgruppe substituiert ist; ein Naphtylrest; ein Phenylrest, der 1- bis 3fach durch Halogenatome, $C_{1-4}$-Alkyl-, $C_{1-4}$-Alkoxygruppen und/oder durch eine Trifluormethyl- oder eine $C_{1-4}$-Acylgruppe substituiert sein kann; ein 6gliedriger Heteroarylrest mit 1 bis 2 Stickstoffatomen.

Besonders bevorzugte Reste für $R^9$ sind ein $C_{1-3}$-Kohlenwasserstoffrest, der durch eine Phenylgruppe substituiert ist, wobei die Phenylgruppe durch eine $C_{1-4}$-Alkoxygruppe substituiert sein kann; ein Naphtylrest; eine Phenylgruppe, die durch 1 oder 2 Halogenatome, $C_{1-4}$-Alkyl-, $C_{1-4}$-Alkoxygruppen und/oder eine Trifluormethylgruppe substituiert sein kann; und die Pyridylgruppe. Besonders wichtig sind eine Benzylgruppe, die durch eine Methoxygruppe substituiert sein kann, eine Phenylgruppe, die durch ein Halogenatom substituiert sein kann, und die Pyridylgruppe. P bedeutet vorzugsweise 2.

Bedeutet $R^4$ eine Gruppe der Formel IV, so sind folgende Bedeutungen für $R^{10}$ bevorzugt:

Ein $C_{1-8}$-Alkylrest, der gegebenenfalls durch einen Phenylrest, der 1 bis 2 $C_{1-4}$-Alkylgruppen und/oder Halogenatome enthalten kann, substituiert ist; ein $C_{3-12}$-Cycloalkylrest, der durch 1 bis 3 Methylgruppen, einen Benzyl- oder einen Phenylrest substituiert sein kann; einen bi- oder tricyclischen $C_{7-10}$-Alkylrest, der gegebenenfalls durch 1 bis 3 Methylreste substituiert ist; oder eine benzokondensierte $C_{5-7}$-Cycloalkylgruppe.

Besonders bevorzugt sind die folgenden Bedeutungen für $R^{10}$: Ein $C_{1-4}$-Alkylrest, der durch einen Phenylrest substituiert ist; ein $C_{6-8}$-Cycloalkylrest, der durch eine Phenylgruppe substituiert sein kann; eine benzokondensierte $C_{5-6}$-Cycloalkylgruppe.

$R^{11}$ bedeutet bevorzugt ein Wasserstoffatom oder einen $C_{1-8}$-Alkylrest und insbesondere ein Wasserstoffatom oder einen $C_{1-4}$-Alkylrest.

Bedeutet $R^4$ eine Gruppe der Formel V, so bedeuten $R^{12}$ und $R^{13}$ bevorzugt Wasserstoff oder Halogen oder $C_{1-4}$-Alkylgruppen und n die Zahl 1 oder 2. Besonders bevorzugt sind Wasserstoffatome für $R^{12}$ und $R^{13}$ und 2 für n.

Bedeutet $R^4$ eine Gruppe der Formel VI, so sind für G ein Sauerstoff- oder Schwefelatom und $R^{14}$ und $R^{15}$ Wasserstoffatome oder Methylgruppen bevorzugt.

Bevorzugt bedeutet $R^4$ eine der Gruppe der Formeln II, III, IV und/oder V, besonders bevorzugt eine der Gruppen II, III und/oder V.

Bei den Verbindungen der Formel I, in denen A, B und $R^2$ für ein Wasserstoffatom steht, handelt es sich um 6-Aryl-4,5-dihydro-3(2H)-pyridazinone, während Verbindungen der Formel I, in denen A, B Wasserstoffatome sind und $R^1$ und $R^2$ zusammen eine Methylen-, Ethylen-, Propylen- oder Butylengruppe bedeuten, als 2-Aryl-3,4-diaza-bicyclo[4.1.0]hepten-(2)-one-(5), 2-Aryl-3,4-diaza-bicyclo[4.2.0]octen-(2)-one-(5), 2-Aryl-3,4-diaza-bicyclo-[4.3.0]nonen-(2)-one-(5) bzw. 2-Aryl-3,4-diaza-bicyclo-[4.40]decen-(2)-one(5) zu bezeichnen sind.

Bei den Verbindungen der Formel I, in denen A und B zusammen eine Bindung bilden, handelt es sich um 6-Aryl-3(2H)pyridazinone.

Als physiologisch verträgliche Säuren kommen beispielsweise Salzsäure, Schwefelsäure, Phosphorsäure, Essigsäure, Zitronensäure, Weinsäure, Malonsäure, Bernsteinsäure, Maleinsäure, Fumarsäure, Äpfelsäure, Milchsäure, Diamidosulfonsäure, Glutaminsäure und Asparaginsäure in Betracht.

Die neuen Verbindungen der Formel I und deren Salze mit physiologisch verträglichen Säuren lassen sich herstellen, indem man

a) eine Verbindung der Formel VII

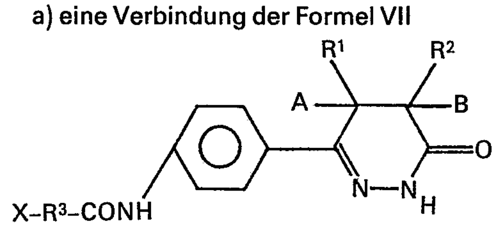

(VII)

in der

X ein Halogenatom ist und

A, B, $R^1$, $R^2$ und $R^3$ die angegebenen Bedeutungen haben, mit einer Verbindung der Formel VIII

$R^4$ - H (VIII),

in der $R^4$ die angegebenen Bedeutungen hat, umsetzt oder

b) ein Aminophenylpyridazinon der Formel IX

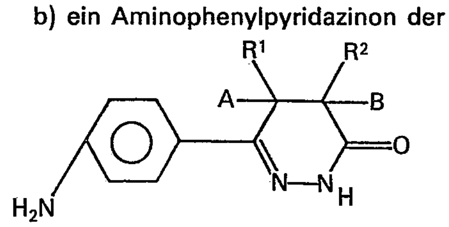

(IX)

in der A, B, $R^1$ und $R^2$ die angegebenen Bedeutungen haben, mit einer Verbindung der Formel X

$R^4$-$R^3$-CO-Y . HY (X),

in der $R^3$ und $R^4$ die angegebenen Bedeutungen haben und Y für ein Chlor- oder Bromatom steht, acyliert oder

c) eine Ketocarbonsäure der Formel XI

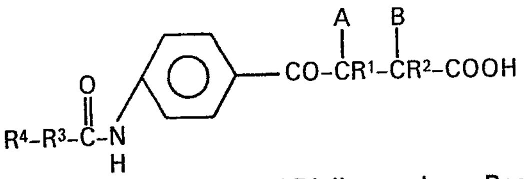

(XI)

in der A, B, $R^1$, $R^2$, $R^3$ und $R^4$ die angebene Bedeutung haben, mit Hydrazin cyclisiert oder

d) falls A, B zusammen eine Bindung bilden und $R^2$ eine Methylgruppe bedeutet, ein Dihydropyridazinon der Formel I

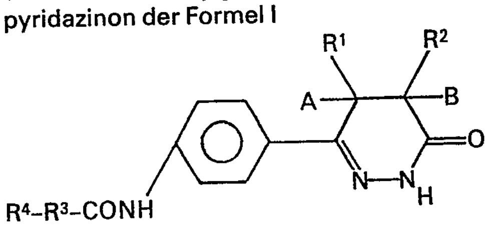

(I),

in der $R^1$, $R^3$ und $R^4$ die oben angegebenen Bedeutungen besitzen und A, B und $R^2$ Wasserstoffatome bedeuten, mit Formaldehyd umsetzt
und die so erhaltenen Verbindungen gegebenenfalls in ihre Salze mit physiologisch verträglichen Säuren überführt.

Die Umsetzung a) wird unter üblichen Bedingungen durchgeführt, d.h. in der Regel unter Verwendung von wenigstens einer äquimoleren Menge der Verbindung der Formel VIII, in Gegenwart von wenigstens einer äquimolaren Menge einer Hilfsbase als säurebindendes Mittel, gegebenenfalls unter Zusatz eines Iodids als Katalysator (wenn X nicht Iod ist), zweckmässig in Gegenwart eines Lösungsmittels, bei Temperaturen von 0 bis 140°C, vorzugsweise 30 bis 100°C, gegebenenfalls bei der Siedetemperatur des Reaktionsgemisches und gegebenenfalls unter Anwendung von Druck. Es kann auch auf den Zusatz einer Hilfsbase verzichtet werden. In einem solchen Fall ist es zweckmässig, wenigstens zwei Äquivalente der Verbindung der Formel VIII einzusetzen.

Als Lösungsmittel kommen unter den Reaktionsbedingungen inerte Lösungsmittel, wie aromatische Kohlenwasserstoffe, beispielsweise Toluol oder Xylol, aliphatische oder aromatische Chlorkohlenwasserstoffe, wie Methylenchlorid, Ethylenchlorid oder Chlorbenzol, cyclische aliphatische Ether, wie Tetrahydrofuran oder Dioxan, niedere Alkanole wie Methanol, Ethanol oder Isopropanol, aliphatische Ketone, wie Aceton, Diethylketon oder Methylethylketon, oder Dialkylamide, wie Dimethylformamid oder N-Methylpyrrolidon, in Betracht. Hilfsbasen als säurebindende Mittel sind zweckmässigerweise anorganische Basen, wie Natrium- oder Kaliumcarbonat, Natrium- oder Kaliumhydrogencarbonat, tertiäre organische Amine, wie Triethylamin, oder ein Überschuss der Amine $R^4H$.

Iodide als Katalysatoren sind zweckmässigerweise Alkaliiodide, bevorzugt Kaliumiodid oder Natriumiodid, oder quartäre Ammoniumiodide, wie z.B. Tetrabutylammoniumiodid. Das Iodid wird in der Regel in Mengen von 1 bis 10 Mol.% verwendet. Es können aber auch bis zu 1-molare Mengen des Katalysators eingesetzt werden.

Die als Ausgangsverbindung verwendeten Substanzen der Formel VII sind bekannt oder lassen sich nach bekannten Methoden herstellen.

Die Umsetzung b) findet unter den für eine N-Acylierung üblichen Bedingungen statt, in der Regel unter Verwendung von wenigstens einer äquimolaren Menge des Säurehalogenids zweckmässig in Gegenwart eines Lösungsmittels, gegebenenfalls in Gegenwart eines säurebindenen Mittels, bei einer Temperatur von 0 bis zu 100°C, bevorzugt bis zu 70°C, gegebenenfalls bei der Siedetemperatur des Reaktionsgemisches und gegebenenfalls unter Anwendung von Druck.

Als Lösungsmittel im technischen Massstab kommen vorzugsweise Ketone; wie Aceton, Diethylketon oder Methylethylketon, im übrigen z.B. aromatische Kohlenwasserstoffe, wie Benzol, Toluol oder Xylol, cyclische Ether, wie Tetrahydrofuran oder Dioxan, aliphatische oder aromatische Chlorkohlenwasserstoffe, wie Methylenchlorid, Ethylenchlorid oder Chlorbenzol, oder Dialkylamide, wie Dimethylformamid oder N-Methylpyrrolidon, in Betracht. Säurebindende Mittel sind zweckmässigerweise schwache anorganische Basen, wie Natrium- oder Kaliumcarbonat, Natrium- oder Kaliumhydrogencarbonat, oder organische Basen, wie tertiäre Amine.

Die als Ausgangsverbindung verwendeten Substanzen der Formel IX sind bekannt oder lassen sich nach bekannten Methoden herstellen.

Die Ringschlussreaktion c) mit Hydrazin, das bevorzugt als Hydrat eingesetzt wird, geschieht vorteilhaft in einem Lösungsmittel, insbesondere einem niederen Alkohol, wie Methanol, Ethanol oder Propanol, einem cyclischen aliphatischen Ether, wie Tetrahydrofuran oder Dioxan, oder in einem Dialkylamid, insbesondere Dimethylformamid oder N-Methylpyrrolidon, wobei die Temperatur 20 bis 150°C, vorzugsweise 50 bis 100°C, betragen kann. In der Regel wird hierbei pro Mol XI 1 Mol Hydrazin bzw. Hydrazinhydrat verwendet.

Die Umsetzung d) wird vorteilhaft in einem Lösungsmittel wie z.B. einem niederen Alkanol, wie Methanol, Ethanol, Isopropanol oder einem Dialkylamid, wie Dimethylformamid, N-Methylpyrrolidon, in Gegenwart einer Base, wie NaOH, KOH oder $K_2CO_3$ oder eines tertiären Amins bei Temperaturen von 20 bis 150°C, vorzugsweise bei 50 bis 100°C, durchgeführt. Zweckmässigerweise verwendet man pro Mol I (A, B, $R^2$ = H) 1 bis 1,5 Mol Formaldehyd. Die Reaktion kann bei Normaldruck oder unter erhöhtem Druck durchgeführt werden.

Die als Ausgangssubstanzen verwendeten Verbindungen der Formel XI lassen sich herstellen, indem man eine Aminosäure der Formel XII

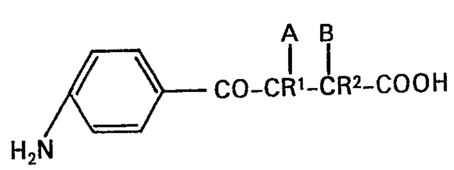

(XII),

in der A, B, $R^1$ und $R^2$ die für Formel I angegebe-

nen Bedeutungen haben, mit einer Verbindung der Formel X unter den für die Acylierung einer Verbindung der Formel IX mit einer Verbindung der Formel X angegebenen Bedingungen acyliert und die dabei erhaltene Verbindung, wenn es sich um das Hydrochlorid oder das Hydrobromid einer Verbindung der Formel XI handelt, in die freie Aminosäure überführt.

Die Verbindungen der Formel XII sind bekannt oder lassen sich nach bekannten Methoden herstellen.

Nach den geschilderten Verfahren lassen sich die in der folgenden Übersicht enthaltenen Verbindungen darstellen.

Verbindungen I, in denen A, B und $R^2$ Wasserstoffatome sind und $R^1$ kein Wasserstoffatom bedeutet (4,5-Dihydro-3(2H)-pyridazinone) weisen ein asymmetrisches Kohlenstoffatom in 5-Stellung auf und werden daher normalerweise in Form ihres Racemats erhalten. Die Erfindung betrifft auch die Enantiomeren, die vorteilhaft bereits auf der Stufe der Verbindung IX, soweit darin A, B und $R^2$ Wasserstoffatome sind, in bekannter Weise, z.B. durch Bildung diastereomerer Salze mit einer optisch aktiven Carbonsäure, wie Dibenzoylweinsäure oder Campher-10-sulfonsäure, getrennt und durch getrennte Umsetzung der so erhaltenen optisch aktiven Vorprodukte gewonnen werden.

Verbindungen der Formel I, in denen $R^3$ eine substituierte Alkylengruppe bedeutet, können ein asymmetrisches Kohlenstoffatom in dieser Alkylenkette enthalten. Die Erfindung betrifft auch die Enantiomeren, die in bekannter Weise, z.B. durch Bildung diastereomerer Salze mit einer optisch aktiven Carbonsäuren oder Sulfonsäure, getrennt oder durch Einsatz entsprechend optisch aktiver Vorprodukte, wie z.B. der Verbindungen der Formeln VII, X oder XI, erhalten werden können.

Ausserdem können die Verbindungen I im Aminteil $R^4$ asymmetrische Kohlenstoffatome besitzen. Ihre Herstellung wird bevorzugt ausgehend von den optisch aktiven Aminen VIII durch Umsetzung mit einer Verbindung VII oder über die Zwischenprodukte X oder XI durchgeführt.

Verbindungen I, in denen $R^1$ und $R^2$ zusammen eine Methylen-, Ethylen-, Propylen- oder Butylengruppe bedeuten (3,4-Diaza-bicyclo[4.1.0]heptenone, 3,4-Diaza-bicyclo[4.2.0]-octenone, 3,4-Diaza-bicyclo[4.3.0]nonenone und 3,4-Diaza-bicyclo[4.4.0]decenone), bilden wegen der asymmetrischen C-Atome 1 und 6 des 3,4-Diaza-bicyclo[4.n.0]alken-(2)-on-(5)-Ringes ebenfalls Racemate. Die Erfindung betrifft auch die hieraus erhältlichen Enantiomeren.

Die erfindungsgemässen Verbindungen der Formel I und ihre Salze mit einer pharmazeutisch verträglichen Säure zeichnen sich insbesondere durch thrombozytenaggregationshemmende sowie blutdrucksenkende, magensekretionshemmende bzw. positiv inotrope Eigenschaften aus.

Zur Untersuchung der pharmakodynamischen Eigenschaften der erfindungsgemässen Produkte wurden folgende Methoden verwendet:

1. Hemmung der durch Collagen induzierten Aggregation von Thrombozyten des Menschen in vitro.

Thrombozytenreiches Plasma wird durch Zentrifugation (300 g, 10 min Dauer bei 4°C) von venösem Citratblut gewonnen. Die photometrische Messung der Thromozytenaggregation erfolgt unter Zusatz von $MgCl_2$ (Endkonzentration 10 mmol/l) und von Collagen Stago (Endkonzentration 0,02 mg/ml) im Born-Aggregometer Mk 3. Als Aggregationsmass findet die maximale Extinktionsänderung/sec Verwendung.

Die Prüfung der aggregationshemmenden Wirksamkeit der Substanzen wird nach einer Inkubationszeit von 10 min vorgenommen.

Als EC 50% wird die Konzentration bestimmt, welche eine 50%ige Hemmung der Aggregation verursacht.

2. Blutdrucksenkende Wirkung an der narkotisierten Ratte.

Zur Testung der blutdrucksenkenden Wirkung erhalten Gruppen von 3 bis 5 männlichen Sprague-Dawley-Ratten (240 bis 280 g) in Urethan-Narkose (1,78 mg/kg i.p.) die Substanzen intraperitoneal oder intravenös appliziert.

Die Messung des Blutdrucks in der Arteria carotis erfolgt über Statham-Transducer. Als ED 20% wird die Dosis bestimmt, welche den mittleren Carotisblutdruck um 20% senkt.

3. Magensektretionshemmung

Die Hemmung der Magensäuresekretion kommt in einem Anstieg des pH-Wertes der Magenschleimhaut zum Ausdruck. Sie wird an Gruppen von 4 weiblichen Sprague-Dawley-Ratten (160 bis 180 g) untersucht. Die Tiere erhalten 48 Stunden kein Futter (Wasser ad libitum) und werden mit unterschiedlichen Dosen der Prüfsubstanzen (p.o.) vorbehandelt. Nach 1 Stunde werden sie mit Hexaborbarbital-Na (46,4 mg/kg i.v.) narkotisiert. Anschliessend wird eine pH-Elektrode (Philips-Peszialelektrode Typ CJP) in den Magen eingeführt und der pH-Wert auf der Oberfläche der Magenschleimhaut gemessen (pH-Wert bei unbehandelten Tieren: 1,40 ± 0,002; N = 200). Aus der linearen Regression zwischen den Logarithmen der applizierten Dosen und der Zunahme des pH-Wertes wird als ED 0,75 die Dosis bestimmt, die im Vergleich zu unbehandelten Kontrolltieren eine pH-Zunahme um durchschnittlich 0,75 bewirkt. Die erfindungsgemässen Substanzen wirken in diesen Tests in sehr niedrigen Dosen.

Gegenstand der vorliegenden Erfindung sind demnach auch therapeutische Mittel oder Zubereitungen, die neben pharmazeutisch üblichen Träger- und Verdünnungsmitteln eine Verbindung der Formel I oder dessen Salz als Wirkstoff enthalten, sowie die Verwendung dieser Verbindungen zu therapeutischen Zwecken.

Die therapeutischen Mittel oder Zubereitungen können mit den üblichen Trägerstoffen oder Verdünnungsmitteln und den üblicherweise verwendeten pharmazeutisch technischen Hilfsstoffen entsprechend der gewünschten Applikationsart mit einer geeigneten Dosierung in bekannter Weise hergestellt werden. Dabei kommen beim Menschen Dosen von 0,1 bis 100 mg in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Supsensionen oder Depotformen.

Zur praktischen Anwendung werden die erfindungsgemäss zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Mais, Stärke, Alginsäure oder Polyvinylpyrrolidon, Bindemitteln, wie Stärke oder Gelantine, Gleitmitteln, wie Magnesiumstearat oder Talkum und/oder Mitteln zur Erzielung eines Depoteffektes, wie Carboxypolymethylen, Carboxymethylcellulose, Celluloseacetatphthalat oder Polyvinyl-acetat, erhalten werden. Die Tabletten können auch aus mehreren Schichten bestehen (vgl. H. Sucker et al, Pharmazeutische Technologie, Thieme-Verlag, Stuttgart 1978).

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die Herstellung der neuen 6-(Acylaminoaryl)-3(2H)-pyridazinone wird durch die folgenden Beispiele näher erläutert:

Beispiel 1

5,8 g (20 mmol) 6-[p-(3-Chlorpropionylamino)phenyl]-4,5-dihydro-3(2H)-pyridazinon wurden mit 3,5 g (25 mmol) Kaliumcarbonat und 2,17 g (25 mmol) Morpholin in 50 ml Dimethylformamid 8 h bei 80°C gerührt. Nach Zugabe von 500 g Eis/Wasser wurde das Kristallisat abgesaugt. Nach Umkristallisieren aus Dimethylformamid/Wasser trocknete man im Hochvakuum bei 50°C und erhielt 5,4 g (82%) 6-[p-3-(1-Morpholino)propionylamino-phenyl]-4,5-dihydro-3(2H)-pyridazinon als farblose Kristalle, Fp. 183–185°C.

Analog Beispiel 1 wurden die Verbindungen der Beispiele 2 bis 104 hergestellt. Wenn die Verbindungen als Öle anfallen, können sie durch Extraktion mit Methylenchlorid und/oder Säulenchromatographie über Kieselgel oder Aluminiumoxid mit Methylenchlorid/Methanol-Gemischen als Laufmittel gereinigt werden.

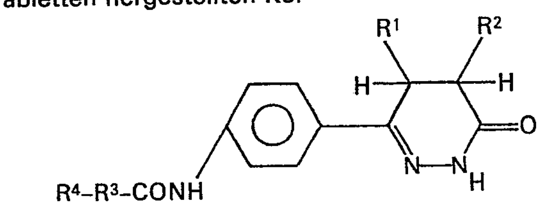

| Beispiel Nr. | $R^4$–$R^3$– | [X $H_2O$] | $R^1$ | $R^2$ | Fp. [°C] |
|---|---|---|---|---|---|
| 2 | $(H_3C)_2N-CH_2CH_2-$ | [1/2 $H_2O$] | $CH_3$ | H | 74–75 |
| 3 | $(H_3C)_2N-CH_2CH_2-$ | | H | H | 168–170 |
| 4 | 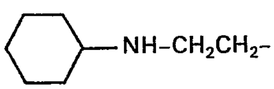 | [1 $H_2O$] | $CH_3$ | H | 143–145 |
| 5 | 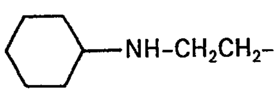 | [1,5 $H_2O$] | H | H | 168–170 |
| 6 | 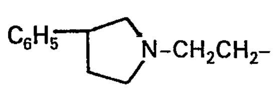 | | $CH_3$ | H | 87–89 |
| 7 | 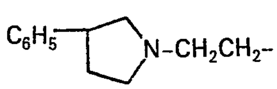 | [1 $H_2O$] | H | H | 165–166 |

| Beispiel Nr. | $R^4$–$R^3$– | [X $H_2O$] | $R^1$ | $R^2$ | Fp. [°C] |
|---|---|---|---|---|---|
| 8 | 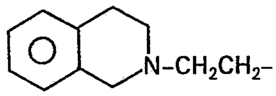 | [1 $H_2O$] | $CH_3$ | H | 119–121 |
| 9 | 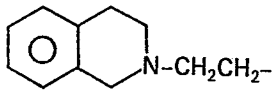 | | H | H | 191–192 |
| 10 | 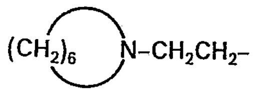 | [1/2 $H_2O$] | $CH_3$ | H | 180–183 |
| 11 | 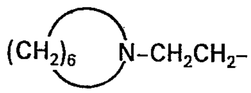 | [1/4 $H_2O$] | H | H | 160–162 |
| 12 | 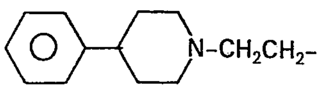 | | $CH_3$ | H | 228–230 |
| 13 | 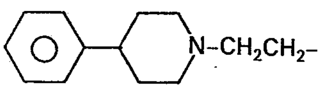 | [1/4 $H_2O$] | H | H | 238–240 |
| 14 | 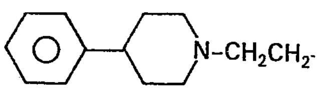 | [1 $H_2O$] | $-CH_2-$ | | 244–247 |
| 15 | 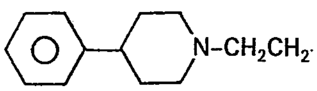 | | $-CH_2-CH_2-$ | | 240–241 |
| 16 | 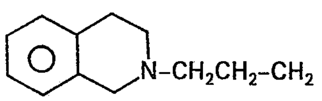 | | $CH_3$ | H | |
| 17 | 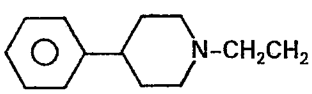 | | $-CH_2-CH_2-CH_2-CH_2$ | | 191–192 |
| 18 | 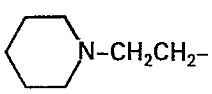 | | $CH_3$ | H | 148–150 |
| 19 | 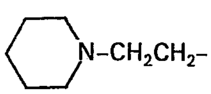 | [1 $H_2O$] | H | H | 150–152 |
| 20 | 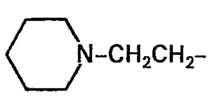 | [1/4 $H_2O$] | $-CH_2-$ | | 154–155 |
| 21 | 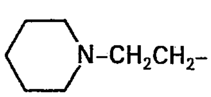 | [1,25 $H_2O$] | | $-CH_2-CH_2-$ | 115–117 |

| Beispiel Nr. | $R^4$–$R^3$– | [X $H_2O$] | $R^1$ | $R^2$ | Fp. [°C] |
|---|---|---|---|---|---|
| 22 | 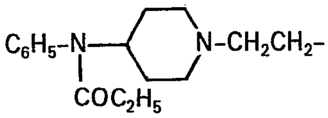 | | $CH_3$ | H | 216–217 |
| 23 | 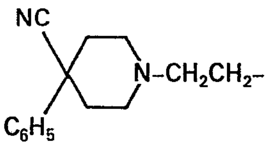 | [$H_2O$] | $CH_3$ | H | 208–210 |
| 24 | 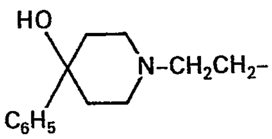 | | $CH_3$ | H | 239–240 |
| 25 | 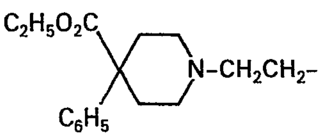 | [$H_2O$] | $CH_3$ | H | 113–115 |
| 26 | 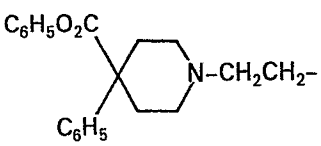 | [$H_2O$] | H | H | 197–199 |
| 27 | 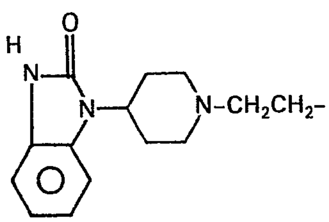 | [1 $H_2O$] | $CH_3$ | H | 168–169 |
| 28 | 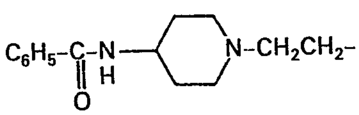 | | $CH_3$ | H | 245–247 |
| 29 | 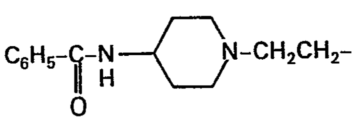 | | H | H | 261–263 |
| 30 | 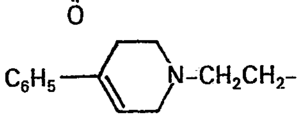 | [1 $H_2O$] | $CH_3$ | H | 204–206 |
| 31 | 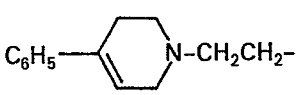 | | H | H | 235–236 |
| 32 | 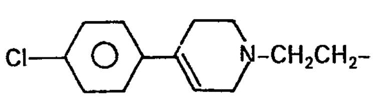 | [1 $H_2O$] | $CH_3$ | H | 200–201 |

| Beispiel Nr. | $R^4$–$R^3$– | [X $H_2O$] | $R^1$ | $R^2$ | Fp. [°C] |
|---|---|---|---|---|---|
| 33 | 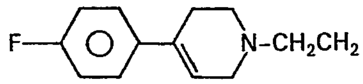 | | $CH_3$ | H | 210–212 |
| 34 | 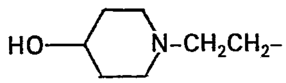 | [1/2 $H_2O$] | $CH_3$ | H | 187–188 |
| 35 | 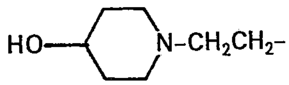 | | H | H | 216–218 |
| 36 | 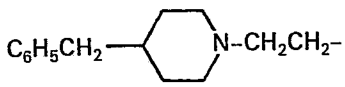 | [1 $H_2O$] | $CH_3$ | H | 190–192 |
| 37 | 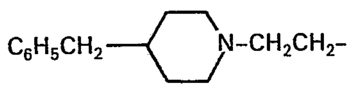 | | H | H | 192–194 |
| 38 | 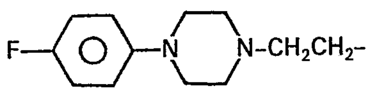 | | $CH_3$ | H | 233–225 |
| 39 | 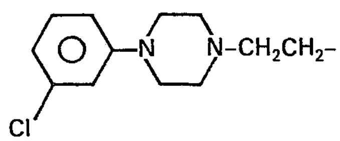 | | $CH_3$ | H | 145–147 |
| 40 | 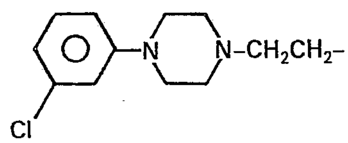 | [1/2 $H_2O$] | H | H | 235–236 |
| 41 | 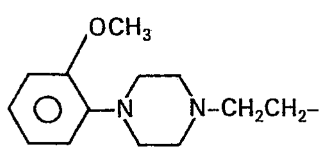 | | H | H | 207–209 |
| 42 | 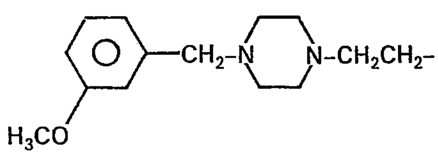 | [1/2 $H_2O$] | $CH_3$ | H | 86–89 |
| 43 | 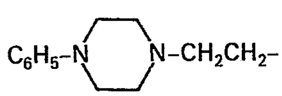 | [1,5 $H_2O$] | $CH_3$ | H | 261–263 |
| 44 | 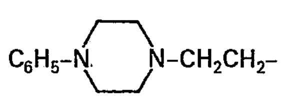 | | H | H | 255–257 |
| 45 | 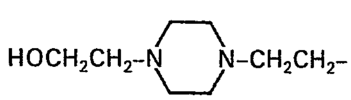 | [1 $H_2O$] | $CH_3$ | H | 228–230 |

| Beispiel Nr. | $R^4$–$R^3$– | [X $H_2O$] | $R^1$ | $R^2$ | Fp. [°C] |
|---|---|---|---|---|---|
| 46 | 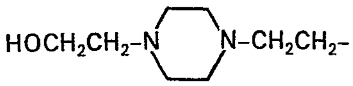 | | H | H | 219–221 |
| 47 | 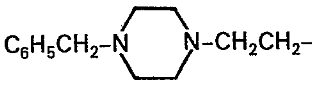 | [1/4 $H_2O$] | $CH_3$ | H | 178–179 |
| 48 | 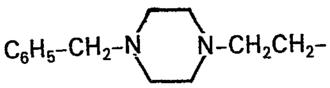 | [1 $H_2O$] | H | H | 171–173 |
| 49 | 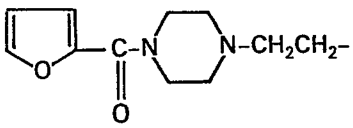 | [1 $H_2O$] | H | H | 134–136 |
| 50 | 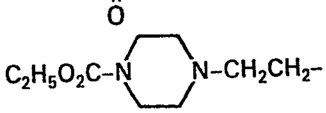 | | H | H | 200–202 |
| 51 | 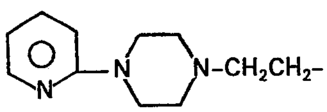 | [1,5 $H_2O$] | $CH_3$ | H | 193–195 |
| 52 | 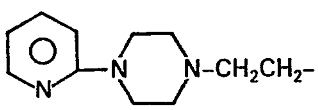 | [1/4 $H_2O$] | H | H | 252–254 |
| 53 | 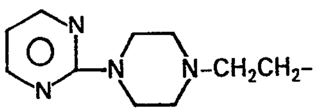 | [1 $H_2O$] | H | H | 224–225 |
| 54 | 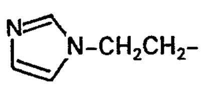 | [1 $H_2O$] | $CH_3$ | H | 198–200 |
| 55 | 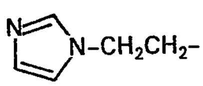 | [1/2 $H_2O$] | H | H | 248–251 |
| 56 | 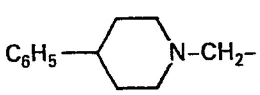 | | $CH_3$ | H | 170–171 |
| 57 | 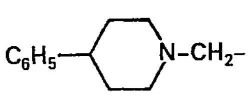 | [1 $H_2O$] | $-CH_2-$ | | 199–200 |
| 58 | 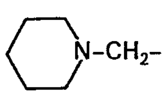 | | $-CH_2-$ | | 184–185 |
| 59 | 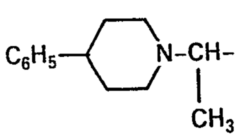 | | $CH_3$ | H | 200–202 |

| Beispiel Nr. | $R^4$–$R^3$– | [X $H_2O$] | $R^1$ | $R^2$ | Fp. [°C] |
|---|---|---|---|---|---|
| 60 | 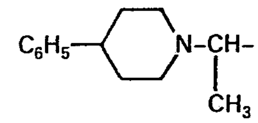 | [1 $H_2O$] | $-CH_2-$ | | 194–195 |
| 61 | 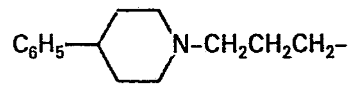 | [1 $H_2O$] | $CH_3$ | H | 112–113 |
| 62 | 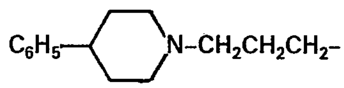 × HCl | [1/2 $H_2O$] | $CH_3$ | H | 295–297 |
| 63 | 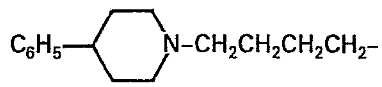 | [1/2 $H_2O$] | H | H | 129–130 |
| 64 | 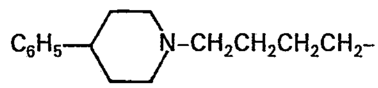 | [1/2 $H_2O$] | $CH_3$ | H | 115–117 |
| 65 | 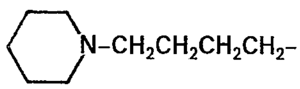 | [1/2 $H_2O$] | H | H | 190–192 |
| 66 | 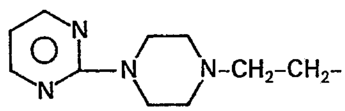 | | $CH_3$ | H | 118–124 |
| 67 | 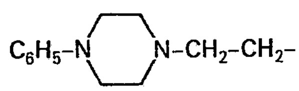 | | $-CH_2-CH_2$ | | |
| 68 | 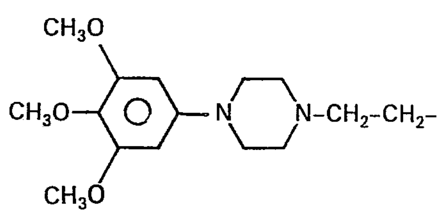 | | $CH_3$ | H | 92–96 |
| 69 | 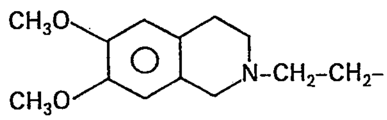 | [3/4 $H_2O$] | H | H | 129–130 |
| 70 | 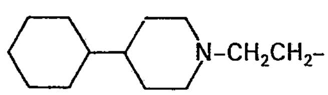 | | H | H | 263–265 |
| 71 | 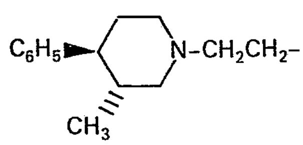 | | H | H | 207–209 |
| 72 | 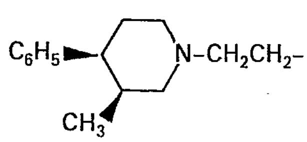 | | H | H | 183–184 |

| Beispiel Nr. | $R^4$–$R^3$– | [X $H_2O$] | $R^1$ | $R^2$ | Fp. [°C] |
|---|---|---|---|---|---|
| 73 | 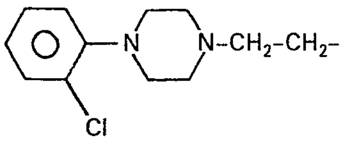 | | $CH_3$ | H | 63–65 |
| 74 | 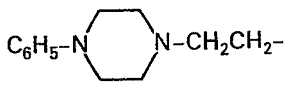 | | $-CH_2-$ | | 265–267 |
| 75 | 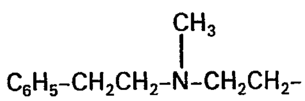 | | H | H | 121–123 |
| 76 | 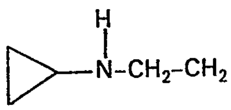 | | $CH_3$ | H | |
| 77 | 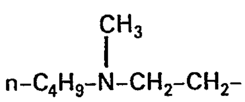 | | $CH_3$ | H | 60–66 |
| 78 | 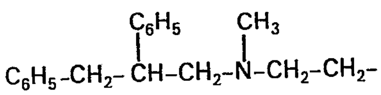 | | $CH_3$ | H | |
| 79 | 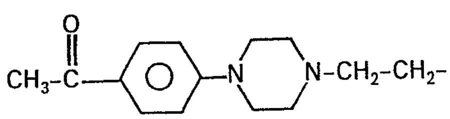 | | $CH_3$ | H | 232–234 |
| 80 | 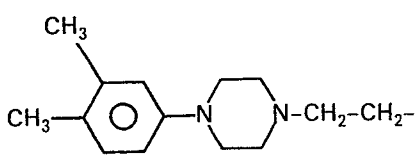 | | $CH_3$ | H | 201–202 |
| 81 | 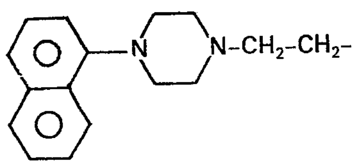 | | $CH_3$ | H | |
| 82 | 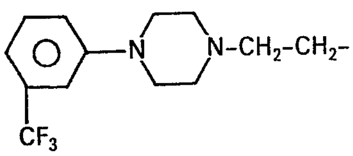 | | $CH_3$ | H | 185–187 |
| 83 | 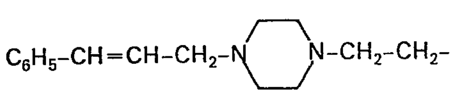 | | $CH_3$ | H | Öl |
| 84 | 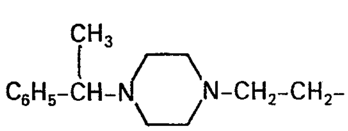 | | $CH_3$ | H | 132–135 |

| Beispiel Nr. | $R^4$–$R^3$– [X $H_2O$] | $R^1$ | $R^2$ | Fp. [°C] |
|---|---|---|---|---|
| 85 | 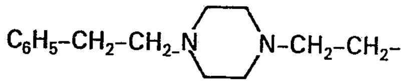 | $CH_3$ | H | 115–120 |
| 86 | 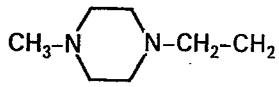 | $CH_3$ | H | Öl |
| 87 | 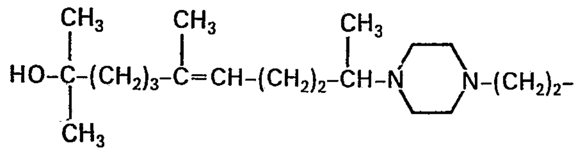 | $CH_3$ | H | Öl |
| 88 | 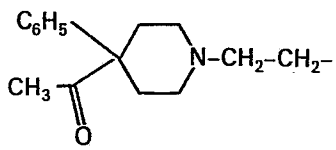 | $CH_3$ | H | 200–202 |
| 89 | 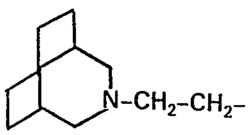 | $CH_3$ | H | 187–189 |
| 90 | 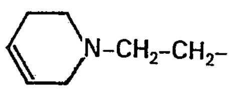 | $CH_3$ | H | 85–87 |
| 91 | 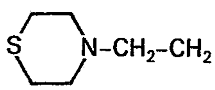 | $CH_3$ | H | 183–185 |
| 92 | 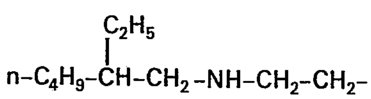 | $CH_3$ | H | Öl |
| 93 | $n-C_{12}H_{25}-NH-CH_2-CH_2-$ | $CH_3$ | H | 123–124 |
| 94 | 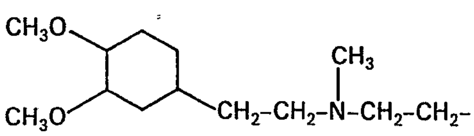 | $CH_3$ | H | 58–60 |
| 95 | 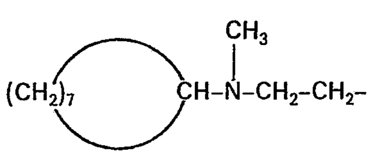 | $CH_3$ | H | 85–87 |

| Beispiel Nr. | $R^4$–$R^3$– | [X $H_2O$] | $R^1$ | $R^2$ | Fp. [°C] |
|---|---|---|---|---|---|
| 96 | 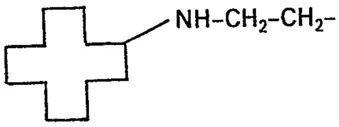 | | $CH_3$ | H | 149–150 |
| 97 | 1-Adamantyl-NH-$CH_2$-$CH_2$- | | $CH_3$ | H | 122–126 |
| 98 | 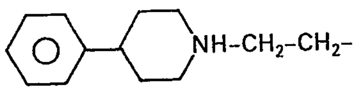 | | $CH_3$ | H | 75–78 |
| 99 | 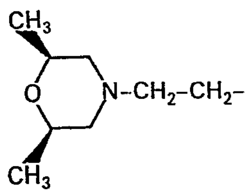 | | $CH_3$ | H | 139–142 |
| 100 | 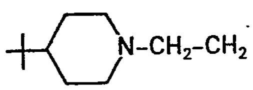 | | H | H | 175–177 |
| 101 | 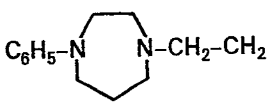 | | $CH_3$ | H | |
| 102 | 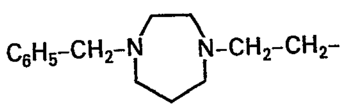 | | $CH_3$ | H | |
| 103 | 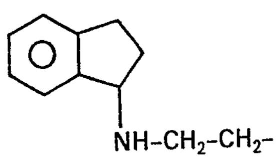 | | $CH_3$ | H | |
| 104 | 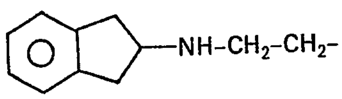 | | $CH_3$ | H | 190–192 |

Analog Beispiel 1 wurden aus 6-[p-(3-Chlorpropionylamino)phenyl]-3-(2H)-pyridazinon und aus 6-[p-(3-Chlorpropionylamino)phenyl]-5-methyl-3(2H)-pyridazinon bzw. aus 6-[p-(3-Chlorpropionylamino)phenyl]-6-methyl-3(2H)-pyridazinon die Verbindungen der Beispiele 108 bis 117 erhalten.

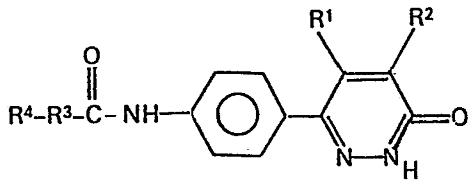

| Beispiel Nr. | $R^4$–$R^3$– | [X $H_2O$] | $R^1$ | $R^2$ | Fp. [°C] |
|---|---|---|---|---|---|
| 105 | 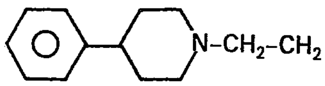 | | H | H | 235–237 |
| 106 | 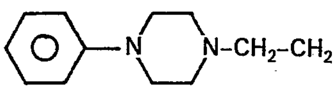 | | H | H | |
| 107 | 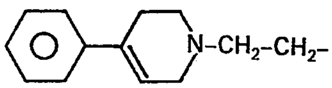 | | H | H | |
| 108 | 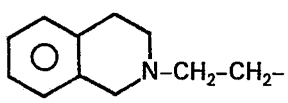 | | H | H | |
| 109 | 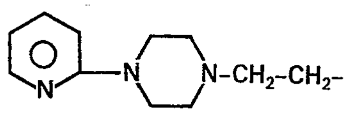 | | H | H | |
| 110 | 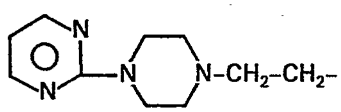 | | H | H | |
| 111 | 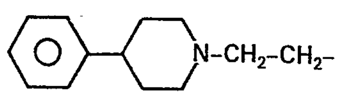 | | $CH_3$ | H | |
| 112 | 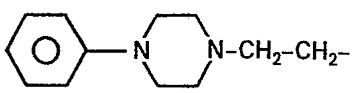 | | $CH_3$ | H | |
| 113 | 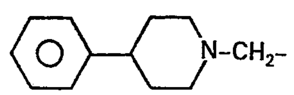 | | H | H | |

Beispiel 114

4,0 g (10 mMol) 6-[p-(3-(4-Phenylpiperid-1-yl)-propionylamino)phenyl]-4,5-dihydro-3(2H)-pyridazinon wurden in 15 ml Ethanol mit 1,5 ml 37%iger Formalinlösung und 40 ml 4% ethanolischer KOH 3 h am Rückfluss gekocht. Nach dem Absaugen der unlöslichen Anteile wurde die Mutterlauge eingeengt und aus Isopropanol umkristallisiert. Man erhielt 2,2 g 6-[p-(3-(4-Phenylpiperid-1-yl)-propionylamino)phenyl]-4-methyl-3(2H)-pyridazinon als hellbeige Kristalle, Fp.: 239–240°C.

Beispiel 115

Analog Beispiel 115 wurde aus 6-[p-(3-(4-Phenylpiperazin - 1 - yl)propionylamino)phenyl] - 4,5-dihydro - 3(2H) - pyridazinon 6 - [p - (3 - (4-Phenylpiperazin-1-yl)propionylamino)phenyl]-4-methyl-3(2H)-pyridazinon hergestellt.

Analog Beispiel 1 wurden durch Umsetzung von 6-[p-(Chlor- oder Bromalkanoyl(amino)-phenyl]-4,5-dihydro-5-methyl-3(2H)pyridazinonen mit 4-Phenylpiperidin bzw. Phenylpiperazin die Verbindungen der Beispiele 116 bis 125 erhalten.

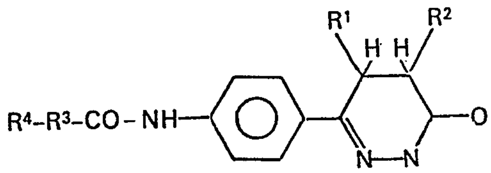

| Beispiel Nr. | $R^4$–$R^3$– | [X $H_2O$] | $R^1$ | $R^2$ | Fp. [°C] |
|---|---|---|---|---|---|
| 116 | 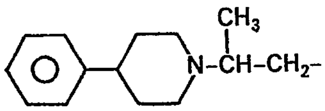 | | $CH_3$ | H | |
| 117 | 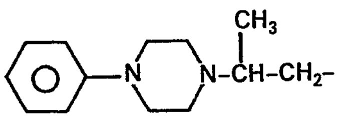 | | $CH_3$ | H | |
| 118 | 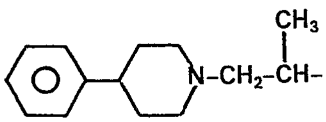 | | $CH_3$ | H | |
| 119 | 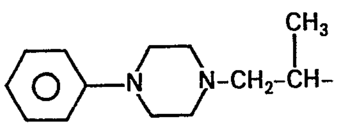 | | $CH_3$ | H | |
| 120 | 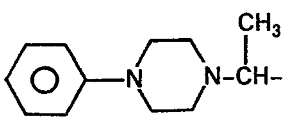 | | $CH_3$ | H | |
| 121 | 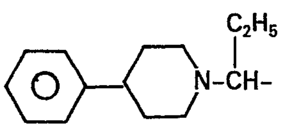 | | $CH_3$ | H | |
| 122 | 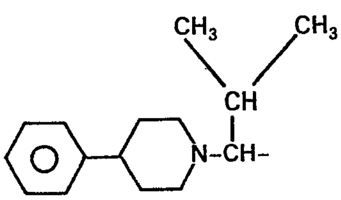 | | $CH_3$ | H | |
| 123 | 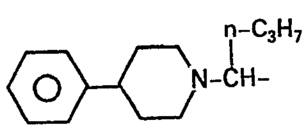 | | $CH_3$ | H | |
| 124 | 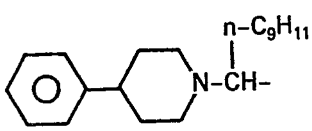 | | $CH_3$ | H | |
| 125 | 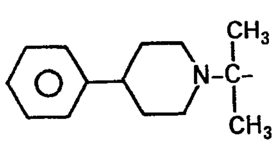 | | $CH_3$ | H | |

Beispiel 126
6-{p-[3-(4-Phenylpiperidino)-propionylamino]phenyl}-4,5-dihydro-3(2H)-pyridazinon

A. 3 - [p - (3 - Chlorpropionylamino)benzoyl]propionsäure

19,2 g (100 mmol) 3-(p-Aminobenzoyl)-propionsäure wurden mit 24,8 g (195 mmol) 3-Chlorpropionylchlorid in 200 ml absolutem Aceton 10 h bei Raumtemperatur gerührt. Nach dem Einengen wurde der Rückstand mit 200 ml Wasser verrührt. Man saugte bei 10°C ab, wusch mit kaltem Aceton und trocknete im Vakuum bei 100°C. Man erhielt 21 g (75%) 3-[p-(3-Chlorpropionylamino)benzoyl]-propionsäure als gelbliche Kristalle, Fp. 181 bis 182°C (Acetonitril).

B. 3p - [3 - (4 - Phenylpiperidino)propionylamino] benzoylpropionsäure

56 g (200 mmol) 3-[p-(3-Chlorpropionylamino)benzoyl]propionsäure wurden mit 60,7 g (440 mmol) Kaliumcarbonat und 35,5 g (220 mmol) Phenylpiperidin in 900 ml Dimethylformamid 8 h bei 80°C gerührt. Nach Zugabe von 2 kg Eis/Wasser wurde das Kristallisat abgesaugt. Nach Umkristallisieren aus Dimethylformamid/Wasser trocknete man im Hochvakuum bei 50°C und erhielt 70 g (86%) 3- {p-[3-(4-Phenylpiperidino)propionylamino]benzoyl}propionsäure, farblose Kristalle, Fp. 237 bis 238°C.

C. 6-{p - [3 - (4 - Phenylpiperidino)propionylamino]phenyl}-4,5-dihydro-3(2H)-pyridazinon

10 g (23,7 mmol) 3-{p-[3-(4-Phenylpiperidino)propionylamino]benzoyl}propionsäure wurden mit 1,2 g (23,7 mmol) Hydrazinhydrat in 200 ml Ethanol 5 h bei 80°C gerührt. Man saugte ab und kristallisierte aus Dimethylformamid/Wasser um. Man erhielt 7,6 g (78%) 6- {p-[3-(4-Phenylpiperidino)propionylamino]phenyl}--4,5-dihydro-3(2H)-pyridazinon. ¼ $H_2O$, farblose Kristalle, Fp. 238 bis 240°C.

Analog Beispiel 126 können die Verbindungen der Beispiel 1 bis 125 hergestellt werden.

Beispiel 127
6-{p-[3-(2-Phenylethylamino)propionylamino]phenyl}-4,5-dihydro-3(2H)-pyridazinon

11,18 g (40 mmol) 6-[p-(3-Chlorpropionylamino)phenyl]4,5-dihydro-3(2H)-pyridazionon wurden in 250 ml Ethanol vorgelegt. Nach dem Zutropfen von 26,5 g (220 mmol) 2-Phenylethylamin wurde 6 h am Rückfluss gekocht. Die unlöslichen Anteile wurden abfiltriert und die Mutterlauge nach Abziehen des Lösungsmittels durch Zugabe von Aceton kristallisiert. Nach zweifachem Umkristallisieren aus Methanol erhielt man 5,3 g 6-{ p-[3-(2-Phenylethylamino)propionylamino]phenyl}-4,5-dihydro-3(2H)-pyridazinon . HCl, Fp. 269–270°C

Zur Überführung in die freie Base wurde das oben erhaltene Hydrochlorid in Methanol gelöst und mit einer äquimoleren Menge Natriummethylat versetzt. Nach Abziehen des Lösungsmittels und Zugabe von $H_20$ wurde die Substanz mit Methylenchlorid extrahiert. Nach Trocknen der Methylenchloridphase über $Na_2SO_4$ und Abziehen des Lösungsmittels wurde die Substanz mit Aceton verrührt und abgesaugt. Man erhielt 6- {p-[3-(2-Phenylethylamino)-propionylamino] phenyl}-4,5-dihydro-3(2H)pyridazinon vom Fp. 130–132°C.

Galenische Beispiele

A) Herstellung von Tabletten

Zusammensetzung:

| | |
|---|---|
| Wirkstoff | 10 mg |
| Polyvinylpyrrolidon (mittl. M.G. 25.000) | 170 mg |
| Polyethylenglykol (mittl. M.G. 4.000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10%iger wässriger Lösung befeuchtet, durch ein Sieb mit der lichten Maschenweite 1,0 mm getrieben und bei 50°C getrocknet. Dieses Granulat wird mit Polyethylenglykol (mittl. M.G. 4.000), Hydroxypropylmethylcellulose, Talkum und Magnesiumstearat vermischt und zu Tabletten à 240 mg verpresst.

B) Herstellung von Dragees:

Zusammensetzung der Drageekerne:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8%igen wässrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpresst. Die erhaltenen Drageekerne werden in üblicher Weise mit einer Hülle überzogen, die im wesentlichen aus Zucker und Talkum besteht.

**Patentansprüche für die Vertragstaaten: BE, CH, DE, FR, GB, IT, LI, NL, SE**

1. 6-Aryl-4,5-dihydro-3(2H)-pyridazinon-derivate der Formel I

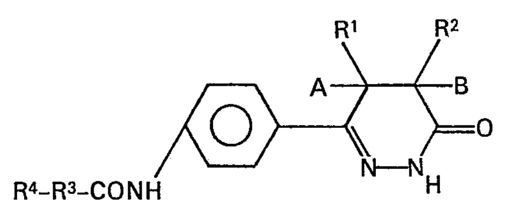
(I)

in der

A und B Wasserstoffatome sind oder gemeinsam eine Bindung bilden,

$R^1$ ein Wasserstoffatom oder eine $C_{1-3}$-Alkylgruppe darstellt,
$R^2$ ein Wasserstoffatom ist, oder – falls A und B Wasserstoffatome sind – $R^1$ und $R^2$ zusammen einen $C_{1-4}$-Alkylenrest bedeuten,
$R^3$ eine geradkettige $C_{1-4}$-Alkylengruppe, die durch 1 bis 2 $C_{1-5}$-Alkylgruppen substituiert sein kann, darstellt und
$R^4$ a) für einen Pyrrol-1-yl-, Imidazol-1-yl-, Pyrazol-1-yl- oder 1,2,4-Triazol-1-yl-Rest steht, oder

b) eine Gruppe der Formel II

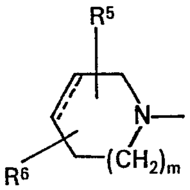

(II)

darstellt, worin
die gestrichelte Linie eine zusätzliche Bindung bedeuten kann,
$R^5$ und $R^6$ gleich oder verschieden sind und Wasserstoffatome, $C_{1-4}$-Kohlenwasserstoffreste, die gegebenenfalls durch eine $C_{3-8}$-Cycloalkylgruppe oder einen Phenylrest, der gegebenenfalls 1 bis 3 $C_{1-4}$-Alkyl-, $C_{1-4}$-Alkoxy-, Trifluormethyl- und/oder Nitrogruppen und/oder Halogenatome aufweist, substituiert sind, $C_{3-8}$-Cycloalkyl-, Hydroxy-, Trifluormethyl-, $C_{1-4}$-Acyl-,Carboxy-, ($C_{1-5}$-Alkoxy)carbonyl, Cyangruppen oder gegebenenfalls 1- bis 3fach durch Halogenatome oder $C_{1-4}$-Alkyl, $C_{1-4}$-Alkoxy-, Hydroxy-, Trifluormethyl-, $C_{1-4}$-Acyl-, Carboxy-, ($C_{1-5}$-Alkoxy)-carbonyl-, Cyan-, Nitro-, Amino-, $C_{1-4}$-Alkylamino, Di-$C_{1-4}$-alkylamino, Anilino- oder $C_{1-4}$-Acylaminogruppen substituierte $C_6$-$C_{10}$-Aryl- oder 5- bis 6gliedrige gegebenenfalls benzokondensierte Heteroarylreste mit 1 bis 3 Heteroatomen bedeuten, oder Gruppen der Formel $R^7R^8N$- darstellen, in der $R^7$ und $R^8$ gleich oder verschieden sind und Wasserstoffatome, $C_{1-8}$-Alkylreste, die gegebenenfalls durch einen Phenylrest, der gegebenenfalls 1 bis 3 $C_{1-4}$-Alkyl-, $C_{1-4}$-Alkoxy-, Trifluormethyl- und/oder Nitrogruppen und/oder Halogenatome aufweist, substituiert sind, Phenylgruppen, die durch 1 bis 3 Halogenatome, $C_{1-4}$-Alkyl, $C_{1-4}$-Alkoxy-, Trifluormethyl-, $C_{1-4}$-Acyl-, Carboxy-, ($C_{1-5}$-Alkoxy)-carbonyl-, Cyan- und/oder Nitrogruppen substituiert sein können, oder $C_{1-8}$-Acyl- oder $C_{6-10}$-Aroylreste bedeuten, wobei die Aroylgruppe 1- bis 3fach durch Halogenatome, $C_{1-4}$-Alkyl-, $C_{1-4}$-Alkoxy-, Hydroxy- Trifluormethyl-, $C_{1-4}$-Alkylthio-, $C_{1-4}$-Alkylsulfoxy-, $C_{1-4}$-Alkylsulfonyl-, $C_{1-4}$-Acyl-, Carboxy-, ($C_{1-5}$-Alkoxy)-carbonyl-, Cyan-, Nitro-, Amino-, $C_{1-4}$-Alkylamino-, Di-$C_{1-4}$-Alkylamino- oder $C_{1-4}$-Acylaminogruppen substituiert sein kann, oder die Gruppe $R^7R^8N$- für einen Benzimidazol-2-on-1-yl-rest steht, oder
$R^5$ und $R^6$ zusammen eine $C_{1-4}$-Alkylenkette bilden, wobei der Bicyclus gegebenenfalls durch 1 bis 3 $C_{1-3}$-Alkylgruppen substituiert ist, und
m = 0, 1, 2 oder 3 ist, oder

c) eine Gruppe der Formel III

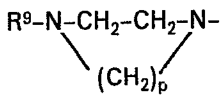

(III)

darstellt, in der $R^9$ einen $C_{1-14}$-Kohlenwasserstoffrest, der gegebenenfalls durch eine Hydroxygruppe, eine $C_{3-8}$-Cycloalkylgruppe, wobei eine $C_{5-8}$-Cycloalkylgruppe benzokondensiert sein kann oder eine $C_{6-10}$-Arylgruppe, die 1 bis 3 $C_{1-4}$-Alkyl-, $C_{1-4}$-Alkoxy-, Trifluormethyl-, Nitro-, Hydroxy-, $C_{1-4}$-Acyl-, Carboxy-, ($C_{1-5}$-Alkoxy)-carbonyl-, Amido-, N-($C_{1-4}$-Alkyl)amido-, N,N-Di-($C_{1-4}$-Alkyl)-amido-, Tri($C_{1-4}$-Alkyl)-silyl-, Cyan-, Amino-, $C_{1-4}$-Alkylamino-, Di-($C_{1-4}$-alkyl)amino-, $C_{1-4}$-Acylaminogruppen und/oder Halogenatome enthalten kann, substituiert ist;
eine $C_6$-$C_{10}$-Aroylgruppe, die gegebenenfalls durch 1 bis 3 $C_{1-4}$-Alkyl-, $C_{1-4}$-Alkoxy-, Trifluormethyl-, Nitro-, Hydroxy-, $C_{1-4}$-Acyl-, Carboxy-, $C_{1-5}$-Alkoxycarbonyl-, NN-Di-($C_{1-4}$-alkyl)-amido-, Cyan-, Di-($C_{1-4}$-alkyl)aminogruppen und/oder Halogenatome substituiert ist;
eine Heteroaroylgruppe mit 5 bis 6 Ringgliedern, die 1 bis 3 Heteroatome enthalten kann und gegebenenfalls benzokondensiert ist; eine $C_{3-12}$-Cycloalkyl-, $C_{1-8}$-Acyl-, ($C_{1-5}$-Alkoxy)-carbonylgruppe oder einen gegebenenfalls 1- bis 3fach durch Halogenatome oder $C_{1-4}$-Alkyl-, $C_{1-4}$-Alkoxy-, Hydroxy-, Trifluormethyl-, $C_{1-4}$-Acyl-, Carboxy-, ($C_{1-5}$ Alkoxy)-carbonyl-, Cyan-, Nitro-, Amino-, $C_{1-4}$-Alkylamino-, Di-$C_{1-4}$-alkylamino-, Arylamino- oder $C_{1-4}$-Acylaminogruppen substituierten $C_{6-10}$-Aryl- oder einen 5- bis 6gliedrigen, gegebenenfalls benzokondensierten Heteroarylrest, der 1 bis 3 Stickstoffatome und gegebenenfalls ein Sauerstoffatom oder ein Schwefelatom enthält, bedeutet, und
p 2 oder 3 ist, oder

d) eine Gruppe der Formel IV

$$-NR^{10}R^{11} \qquad (IV),$$

in der $R^{10}$ und $R^{11}$ Wasserstoffatome, $C_{1-12}$-Kohlenwasserstoffreste, die gegebenenfalls durch einen oder zwei Phenylreste, die gegebenenfalls 1 bis 3 $C_{1-4}$-Alkyl-, $C_{1-4}$-Alkoxy-, Trifluormethyl- und/oder Nitrogruppen und/oder Halogenatome aufweisen,
eine Naphthylgruppe, eine $C_{3-8}$-Cycloalkylgruppe, die 1 bis 3 $C_{1-4}$-Alkylgruppen enthalten kann, wobei $C_{5-8}$-Cycloalkylgruppen benzokondensiert sein können, oder eine $C_{7-10}$-Bicycloalkylgruppe, die 1 bis 3 $C_{1-4}$-Alkylgruppen enthalten kann, substituiert sind;
$C_{3-12}$-Cycloalkylgruppen, die durch 1 bis 3 $C_{1-4}$-Kohlenwasserstoffreste, die eine Phenylgruppe, die 1- bis 3fach durch $C_{1-4}$-Alkyl-, $C_{1-4}$-Alkoxy-, Trifluormethyl-, Nitril-, Nitrogruppen und/oder Halogenatome substituiert sein kann, oder eine $C_{3-6}$-Cycloalkylgruppe enthalten können, oder durch eine Phenylgruppe, die 1 bis 3 $C_{1-4}$-Alkyl-, $C_{1-4}$-Alkoxy-, Trifluormethyl-, Nitril-, Nitrogruppen und/oder Halogenatome enthalten kann, oder eine $C_{3-8}$-Cycloalkylgruppe substituiert sein können;

$C_{7-10}$ bi- oder tricyclische Alkylreste, die durch 1 bis 3 $C_{1-4}$-Alkylgruppen substituiert sein können, oder

benzokondensierte $C_{5-7}$-Cycloalkylgruppen bedeuten, oder

e) eine Gruppe der Formel V

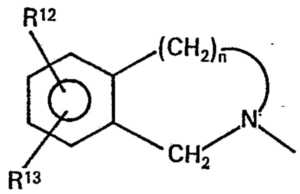

(V)

darstellt, in der $R^{12}$ und $R^{13}$ Wasserstoffatome, Halogenatome, $C_{1-4}$-Alkyl- oder $C_{1-4}$-Alkoxyreste bedeuten, und n für 1, 2 oder 3 steht, oder

f) eine Gruppe der Formel VI

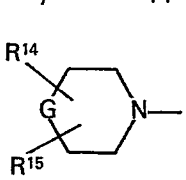

(VI)

in der G ein Sauerstoff- oder Schwefelatom und $R^{14}$ und $R^{15}$ Wasserstoffatome oder $C_{1-4}$-Alkylgruppen bedeuten, darstellt, mit der Massgabe, dass

wenn $R^1$, $R^2$, A und B Wasserstoffatome bedeuten, $R^3$ keine gegebenenfalls durch eine $C_1$-$C_5$-Alkylgruppe substituierte Methylengruppe bedeuten soll, und

wenn $R^2$, A und B Wasserstoffatome bedeuten, $R^1$ ein Wasserstoffatom oder eine $C_1$-$C_3$-Alkylgruppe bedeutet und $R^3$ eine gegebenenfalls durch einen $C_1$-$C_4$-Alkylrest substituierte Methylengruppe bedeutet, $R^4$ nicht eine Gruppe der Formel IV darstellen soll, in der $R^{10}$ und $R^{11}$ Wasserstoffatome oder unsubstituierte $C_1$-$C_4$-Alkylgruppen bedeuten,

sowie deren Salze mit physiologisch verträglichen Säuren.

6-(Acylaminoaryl)-3(2H)-pyridazinon-derivate der Formel I gemäss Anspruch 1, in der

A und B Wasserstoffatome sind oder zusammen eine Bindung bilden,

$R^1$ ein Wasserstoffatom oder eine Methylgruppe bedeutet,

$R^2$ ein Wasserstoffatom ist oder falls A und B Wasserstoffatome sind, $R^1$ und $R^2$ zusammen eine $C_{1-2}$-Alkylengruppe bilden,

$R^3$ eine geradkettige $C_1$-$C_4$-Alkylengruppe, die durch eine $C_1$-$C_3$-Alkylgruppe oder zwei Methylgruppen substituiert sein kann,

$R^4$ a) eine Imidazol-1-yl-Gruppe oder

b) eine Gruppe der Formel II bedeutet, worin $R^5$ ein Wasserstoffatom, eine $C_1$-$C_4$-Alkylgruppe, die gegebenenfalls durch einen Phenylrest substituiert ist, eine $C_3$-$C_6$-Cycloalkylgruppe, eine Phenylgruppe, die gegebenenfalls durch 1 oder 2 $C_1$-$C_4$-Alkyl-, $C_1$-$C_4$-Alkoxygruppen oder Halogenatome und/oder durch eine Nitril-, Nitro- oder Trifluormethylgruppe substituiert sein kann, oder eine Gruppe der Formel $R^7R^8N$ bedeutet, in der $R^7$ ein Wasserstoffatom oder eine Phenylgruppe, die 1- bis 2fach durch Halogenatome, $C_1$-$C_4$-Alkyl-, $C_1$-$C_4$-Alkoxygruppen und/oder eine Nitril- und/oder Nitrogruppe substituiert sein kann, und $R^8$ ein $C_1$-$C_4$-Acyl- oder Benzoylgruppe bedeutet oder $R^7$ und $R^8$ zusammen mit dem Stickstoffatom eine Benzimidazol-2-on-1-yl-Gruppe bilden, $R^6$ ein Wasserstoffatom, eine $C_1$-$C_4$-Alkyl-, eine Hydroxy-, eine $C_1$-$C_4$-Acyl-, eine ($C_1$-$C_5$-Alkoxy)-carbonyl- oder eine Cyangruppe bedeutet oder $R^5$ und $R^6$ gemeinsam eine $C_1$-$C_4$-Alkylenkette bilden, wobei der gebildete Bicyclus durch 1 bis 3 Methylgruppen substituiert sein kann,

m für 0, 1 oder 2 steht, oder

c) eine Gruppe der Formel III bedeutet, in der $R^9$ einen $C_1$-$C_3$-Kohlenwasserstoffrest, der durch einen Napthylrest oder einem Phenylrest substituiert ist, wobei der Phenylrest gegebenenfalls 1 bis 2 Halogenatome, $C_1$-$C_4$-Alkyl-, $C_1$-$C_4$-Alkoxygruppen oder eine Trifluormethyl-, Nitro-, Hydroxy-, $C_1$-$C_4$-Acyl-, ($C_1$-$C_5$-Alkoxy)carbonyl- und/oder Cyangruppe enthalten kann, einen Naphthylrest, einen Phenylrest, der 1- bis 3fach durch Halogenatome, $C_1$-$C_4$-Alkyl-, $C_1$-$C_4$-Alkoxygruppen und/oder eine Trifluormethyl- oder eine $C_1$-$C_4$-Acylgruppe substituiert sein kann, einen 6gliedrigen Heteroarylrest mit 1 bis 2 Stickstoffatomen bedeutet,

p die Zahl 2 bedeutet, oder

d) $R^4$ eine Gruppe der Formel IV bedeutet, in der $R^{10}$ einen $C_1$-$C_8$-Alkylrest, der gegebenenfalls durch einen Phenylrest, der 1 bis 2 $C_1$-$C_4$-Alkylgruppen und/oder Halogenatome enthalten kann, substituiert ist, eine $C_3$-$C_{12}$-Cycloalkylgruppe, die durch 1 bis 3 Methylgruppen, einen Phenyl- oder Benzylrest substituiert sein kann, oder einen $C_7$-$C_{10}$bi- oder tricyclischen Alkylrest, der durch 1 bis 3 Methylreste substituiert sein kann, eine benzokondensierte $C_5$-$C_7$-Cycloalkylgruppe bedeutet und $R^{11}$ ein Wasserstoffatom oder einen $C_1$-$C_8$-Alkylrest bedeutet, der

e) eine Gruppe der Formel V bedeutet, in der $R^{12}$ und $R^{13}$ Wasserstoff- oder Halogenatome oder $C_{1-4}$-Alkylgruppen bedeuten und n 1 oder 2 ist, oder

f) eine Gruppe der Formel VI bedeutet, in der G ein Sauerstoff- oder Schwefelatom und $R^{14}$ und $R^{15}$ Wasserstoffatome oder Methylgruppen darstellen,

mit der Massgabe, dass, wenn $R^1$, $R^2$, A und B Wasserstoffatome bedeuten, $R^3$ keine, gegebenenfalls durch eine $C_1$-$C_3$-Alkylgruppe substituierte Methylengruppe bedeutet und wenn $R^2$, A und B Wasserstoffatome, $R^1$ ein Wasserstoffatom oder eine Methylgruppe und $R^3$ eine gegebenenfalls durch eine $C_1$-$C_3$-Alkylgruppe substituierte Methylengruppe bedeutet, $R^4$ nicht eine Gruppe der Formel IV darstellen soll, in der $R^{10}$ und $R^{11}$ Wasserstoffatome oder unsubstituierte $C_1$-$C_4$-Alkylgruppen bedeuten, sowie deren Salze mit physiologisch verträglichen Säuren.

3. 6-{p-[3-(4-Phenyl-1-piperazino)-propionylamino]-phenyl}-5-methyl-4,5-dihydro-3(2H)-pyridazinon.

4. 6-{p-[3-(4-Pyrid-2-yl-1-piperazino)-propionylamino]-phenyl}-5-methyl-4,5-dihydro-3(2H)-pyridazinon.

5. 6-{p-[3-(4-Benzyl-1-piperazino)-propionylamino]-phenyl}-5-methyl-4,5-dihydro-3(2H)-pyridazinon.

6. 6-{p-[2-(4-Phenxl-1-piperidino)-acetylamino] - phenyl}- 5 - methyl-4,5-dihydro-3(2H)-pyridazinon.

7. 6-{p-[3-(4-Phenyl-1-piperidino)-propionylamino]-phenyl}-4,5-dihydropyridazinon.

8. 6-{p-[3-(4-Phenyl-1-piperidino)-propionylamino]-phenyl}-5-methyl-4,5-dihydropyridazinon.

9. 2-{p-[3-(4-Phenyl-1-piperidino)-propionylamino]-phenyl}-3,4-diazabicyclo[4.1.0]hepten-(2)-on-(5).

10. 6-{p-[3-(4-Phenyl-1-(1,2,3,6-tetrahydropyridino)-propionylamino-]-phenyl}-5-methyl-4,5-dihydro-3(2H)-pyridazinon.

11. 6-{p-[3-(4-Phenyl-1-piperidino)propionylamino]-phenyl}-3-(2H)-pyridazinon.

12. 6-{p-[3-(1-(1,2,3,4-Tetrahydroisochinolino)-propionylamino]-phenyl}-5-methyl-4,5-dihydro-3(2H)pyridazinon.

13. Verfahren zur Herstellung der Verbindungen der Formel I gemäss Anspruch 1, dadurch gekennzeichnet, dass man

a) eine Verbindung der Formel VII

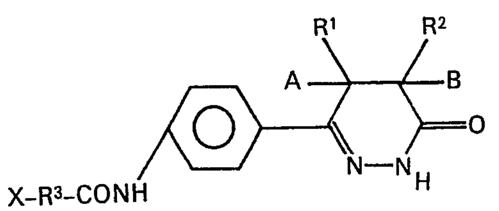
(VII)

in der
X ein Halogenatom ist und
A, B, $R^1$, $R^2$ und $R^3$ die angegebenen Bedeutungen haben, mit einer Verbindung der Formel VIII

$R^4$ - H (VIII)

in der $R^4$ die angegebenen Bedeutungen hat, umsetzt oder

b) ein Aminophenylpyridazinon der Formel IX,

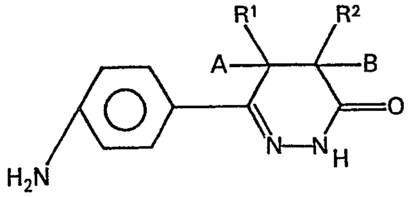
(IX)

in der A, B, $R^1$ und $R^2$ die angegebenen Bedeutungen haben, mit einer Verbindung der Formel X

$R^4$ - $R^3$ - CO - Y . HY (X)

in der $R^3$ und $R^4$ die angegebenen Bedeutungen haben und Y für ein Chlor- oder Bromatom steht, in an sich bekannter Weise acyliert oder

c) eine Ketocarbonsäure der Formel XI

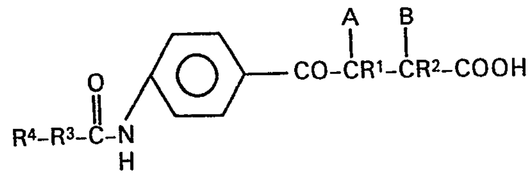
(XI)

in der A, B, $R^1$, $R^2$, $R^3$ und $R^4$ die angegebene Bedeutung haben und die Acylaminogruppe in para- oder meta-Position steht, mit Hydrazin cyclisiert oder

d) falls A, B zusammen eine Bindung bilden und $R^2$ eine Methylgruppe bedeutet, ein Dihydropyridazinon der Formel I

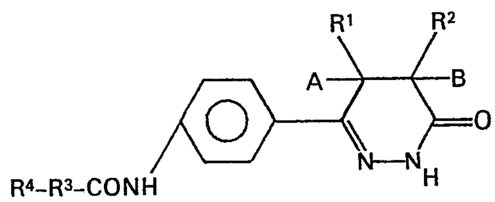
(I),

in der $R^1$, $R^3$ und $R^4$ die oben angebenen Bedeutungen besitzen und A, B und $R^2$ Wasserstoffatome bedeuten, mit Formaldehyd umsetzt und die so erhaltenen Verbindungen gegebenenfalls in ihre Salze mit physiologisch verträglichen Säuren überführt.

14. 6-(Acylaminoaryl)-3(2H)-pyridazinonderivate der Formel I gemäss Anspruch 1 zur Bekämpfung von Krankheiten.

**Patentansprüche für den Vertragstaat: AT**

1. Verfahren zur Herstellung der 6-Aryl-4,5-dihydro-3(2H)-pyridazinon-derivate der Formel I

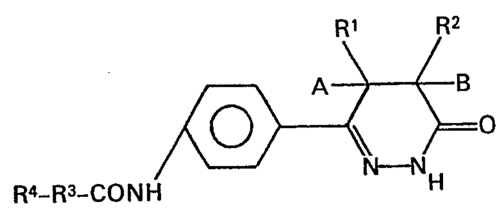
(I),

in der
A und B Wasserstoffatome sind oder gemeinsam eine Bindung bilden,
$R^1$ ein Wasserstoffatom oder eine $C_{1-3}$-Alkylgruppe darstellt,
$R^2$ ein Wasserstoffatom ist oder - falls A und B Wasserstoffatome sind - $R^1$ und $R^2$ zusammen einen $C_{1-4}$-Alkylenrest bedeuten,
$R^3$ eine geradkettige $C_{1-4}$-Alkylengruppe, die durch 1 bis 2 $C_{1-5}$-Alkylgruppen substituiert sein kann, darstellt und
$R^4$ a) für einen Pyrrol-1-yl-, Imidazol-1-yl-, Pyrazol-1-yl- oder 1,2,4-Triazol-1-yl-Rest steht, oder
b) eine Gruppe der Formel II

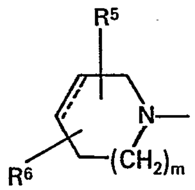

(II)

darstellt, worin
die gestrichelte Linie eine zusätzliche Bindung bedeuten kann,
$R^5$ und $R^6$ gleich oder verschieden sind und Wasserstoffatome, $C_{1-4}$-Kohlenwasserstoffreste, die gegebenenfalls durch eine $C_{3-8}$-Cycloalkylgruppe oder einen Phenylrest, der gegebenenfalls 1 bis 3 $C_{1-4}$-Alkyl-, $C_{1-4}$-Alkoxy-, Trifluormethyl- und/oder Nitrogruppen und/oder Halogenatome aufweist, substituiert sind, $C_{3-8}$-Cycloalkyl-, Hydroxy-, Trifluormethyl-, $C_{1-4}$-Acyl-, Carboxy-, ($C_{1-5}$-Alkoxy)carbonyl, Cyangruppen oder gegebenenfalls 1- bis 3fach durch Halogenatome oder $C_{1-4}$-Alkyl, $C_{1-4}$-Alkoxy, Hydroxy-, Trifluormethyl-, $C_{1-4}$-Acyl-, Carboxy-, ($C_{1-5}$-Alkoxy)-carbonyl-, Cyan-, Nitro-, Amino-, $C_{1-4}$-Alkylamino, Di-$C_{1-4}$-alkylamino, Anilino- oder $C_{1-4}$-Acylaminogruppen substituierte $C_6$-$C_{10}$-Aryl- oder 5- bis 6gliedrige gegebenenfalls benzokondensierte Heteroarylreste mit 1 bis 3 Heteroatomen bedeuten, oder
Gruppen der Formel $R^7R^8N$- darstellen, in der $R^7$ und $R^8$ gleich oder verschieden sind und Wasserstoffatome, $C_{1-8}$-Alkylreste, die gegebenenfalls durch einen Phenylrest, der gegebenenfalls 1 bis 3 $C_{1-4}$-Alkyl-, $C_{1-4}$-Alkoxy-, Trifluormethyl- und/oder Nitrogruppen und/oder Halogenatome aufweist, substituiert sind, Phenylgruppen, die durch 1 bis 3 Halogenatome, $C_{1-4}$-Alkyl, $C_{1-4}$-Alkoxy-, Trifluormethyl-, $C_{1-4}$-Acyl-, Carboxy-, ($C_{1-5}$-Alkoxy)-carbonyl-, Cyan- und/oder Nitrogruppen substituiert sein können, oder $C_{1-8}$-Acyl- oder $C_{6-10}$-Aroylreste bedeuten, wobei die Aroylgruppe 1- bis 3fach durch Halogenatome, $C_{1-4}$-Alkyl-, $C_{1-4}$-Alkoxy-, Hydroxy-, Trifluormethyl-, $C_{1-4}$-Alkylthio-, $C_{1-4}$-Alkylsulfoxy-, $C_{1-4}$-Alkylsulfonyl-, $C_{1-4}$-Acyl-, Carboxy-, ($C_{1-5}$-Alkoxy)-carbonyl-, Cyan-, Nitro-, Amino-, $C_{1-4}$-Alkylamino-, Di-$C_{1-4}$-Alkylamino- oder $C_{1-4}$-Acylaminogruppen substituiert sein kann, oder die Gruppe $R^7R^8N$- für einen Benzimidazol-2-on-1-yl-rest steht, oder
$R^5$ und $R^6$ zusammen eine $C_{1-4}$-Alkylenkette bilden, wobei der Bicyclus gegebenenfalls durch 1 bis 3 $C_{1-3}$-Alkylgruppen substituiert ist, und
m = 0, 1, 2 oder 3 ist, oder
c) eine Gruppe der Formel III

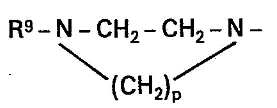

(III)

darstellt, in der $R^9$ einen $C_{1-14}$-Kohlenwasserstoffrest, der gegebenenfalls durch eine Hydroxygruppe, eine $C_{3-8}$-Cycloalkylgruppe, wobei eine $C_{5-8}$-Cycloalkylgruppe benzokondensiert sein kann oder eine $C_{6-10}$-Arylgruppe, die 1 bis 3 $C_{1-4}$-Alkyl-, $C_{1-4}$-Alkoxy-, Trifluormethyl-, Nitro-, Hydroxy-, $C_{1-4}$-Acyl-, Carboxy-, ($C_{1-5}$-Alkoxy)-carbonyl-, Amido-, N-($C_{1-4}$-Alkyl)amido-, N,N-Di-($C_{1-4}$-Alkyl)-amido-, Tri($C_{1-4}$-Alkyl)-silyl-, Cyan-, Amino-, $C_{1-4}$-Alkylamino-, Di-($C_{1-4}$-alkyl)amino-, $C_{1-4}$-Acylaminogruppen und/oder Halogenatome enthalten kann, substituiert ist;
eine $C_6$-$C_{10}$-Aroylgruppe, die gegebenenfalls durch 1 bis 3 $C_{1-4}$-Alkyl-, $C_{1-4}$-Alkoxy-, Trifluormethyl-, Nitro-, Hydroxy-, $C_{1-4}$-Acyl-, Carboxy-, $C_{1-5}$-Alkoxycarbonyl-, NN-Di-($C_{1-4}$-alkyl)-amido-, Cyan-, Di-($C_{1-4}$-alkyl)aminogruppen und/oder Halogenatome substituiert ist;
eine Heteroaroylgruppe mit 5 bis 6 Ringgliedern, die 1 bis 3 Heteroatome enthalten kann und gegebenenfalls benzokondensiert ist; eine $C_{3-12}$-Cycloalkyl-, $C_{1-8}$-Acyl-, ($C_{1-5}$-Alkoxy)-carbonylgruppe oder einen gegebenenfalls 1- bis 3fach durch Halogenatome oder $C_{1-4}$-Alkyl-, $C_{1-4}$-Alkoxy-, Hydroxy-, Trifluormethyl-, $C_{1-4}$-Acyl-, Carboxy-, ($C_{1-5}$-Alkoxy)-carbonyl-, Cyan-, Nitro-, Amino-, $C_{1-4}$-Alkylamino-, Di-$C_{1-4}$-alkylamino-, Arylamino- oder $C_{1-4}$-Acylaminogruppen substituierten $C_{6-10}$-Aryl- oder einen 5- bis 6gliedrigen, gegebenenfalls benzokondensierten Heteroarylrest, der 1 bis 3 Stickstoffatome und gegebenenfalls ein Sauerstoffatom oder ein Schwefelatom enthält, bedeutet, und
p 2 oder 3 ist, oder
d) eine Gruppe der Formel IV

$$-NR^{10}R^{11} \quad (IV),$$

in der $R^{10}$ und $R^{11}$ Wasserstoffatome, $C_{1-12}$-Kohlenwasserstoffreste, die gegebenenfalls durch einen oder zwei Phenylreste, die gegebenenfalls 1 bis 3 $C_{1-4}$-Alkyl-, $C_{1-4}$-Alkoxy-, Trifluormethyl- und/oder Nitrogruppen und/oder Halogenatome aufweisen,
eine Naphthylgruppe, eine $C_{3-8}$-Cycloalkylgruppe, die 1 bis 3 $C_{1-4}$-Alkylgruppen enthalten kann, wobei $C_{5-8}$-Cycloalkylgruppen benzokondensiert sein können, oder eine $C_{7-10}$-Bicycloalkylgruppe, die 1 bis 3 $C_{1-4}$-Alkylgruppen enthalten kann, substituiert sind;
$C_{3-12}$-Cycloalkylgruppen, die durch 1 bis 3 $C_{1-4}$-Kohlenwasserstoffreste, die eine Phenylgruppe, die 1- bis 3fach durch $C_{1-4}$-Alkyl-, $C_{1-4}$-Alkoxy-, Trifluormethyl-, Nitril-, Nitrogruppen und/oder Halogenatome substituiert sein kann, oder eine $C_{3-6}$-Cycloalkylgruppe enthalten können, oder durch eine Phenylgruppe, die 1 bis 3 $C_{1-4}$-Alkyl-, $C_{1-4}$-Alkoxy-, Trifluormethyl-, Nitril-, Nitrogruppen und/oder Halogenatome enthalten kann, oder eine $C_{3-8}$-Cycloalkylgruppe substituiert sein können;
$C_{7-10}$ bi- oder tricyclische Alkylreste, die durch 1 bis 3 $C_{1-4}$-Alkylgruppen substituiert sein können, oder
benzokondensierte $C_{5-7}$-Cycloalkylgruppen bedeuten, oder
e) eine Gruppe der Formel V

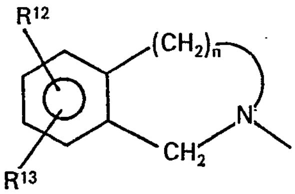

(V)

darstellt, in der $R^{12}$ und $R^{13}$ Wasserstoffatome, Halogenatome, $C_{1-4}$-Alkyl- oder $C_{1-4}$-Alkoxyreste bedeuten, und n für 1, 2 oder 3 steht, oder

f) eine Gruppe der Formel VI

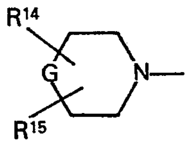

(VI)

in der G ein Sauerstoff- oder Schwefelatom und $R^{14}$ und $R^{15}$ Wasserstoffatome oder $C_{1-4}$-Alkylgruppen bedeuten, darstellt, mit der Massgabe, dass,

wenn $R^1$, $R^2$, A und B Wasserstoffatome bedeuten und die Acylaminogruppe in para-Position am Phenylring in I steht, $R^3$ keine gegebenenfalls durch eine $C_1$-$C_5$-Alkylgruppe substituierte Methylengruppe bedeuten soll, und

wenn $R^2$, A und B Wasserstoffatome bedeuten, $R^1$ ein Wasserstoffatom oder eine $C_1$-$C_3$-Alkylgruppe bedeutet und $R^3$ eine gegebenfalls durch einen $C_1$-$C_4$-Alkylrest substituierte Methylengruppe bedeutet, $R^4$ nicht eine Gruppe der Formel IV darstellen soll, in der $R^{10}$ und $R^{11}$ Wasserstoffatome oder unsubstituierte $C_1$-$C_4$-Alkylgruppen bedeuten,

sowie deren Salze mit physiologisch verträglichen Säuren, dadurch gekennzeichnet, dass man

a) eine Verbindung der Formel VII

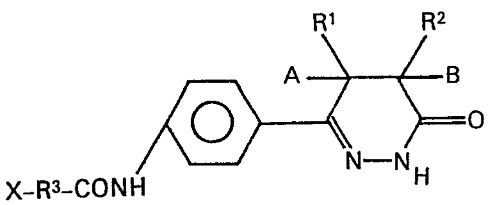

(VII)

in der

X ein Halogenatom ist und

A, B, $R^1$, $R^2$ und $R^3$ die angegebenen Bedeutungen haben, mit einer Verbindung der Formel VIII

$R^4$ - H (VIII)

in der $R^4$ die angegebenen Bedeutungen hat, umsetzt oder

b) ein Aminophenylpyridazinon der Formel IX,

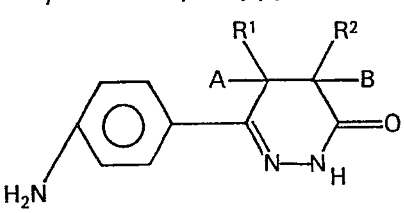

(IX)

in der A, B, $R^1$ und $R^2$ die angegebenen Bedeutungen haben, mit einer Verbindung der Formel X

$R^4$ - $R^3$ - CO - Y . HY (X)

in der $R^3$ und $R^4$ die angegebenen Bedeutungen haben und Y für ein Chlor- oder Bromatom steht, in an sich bekannter Weise acyliert oder

c) eine Ketocarbonsäure der Formel XI

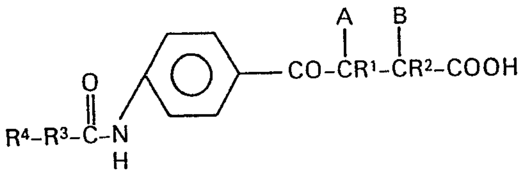

(XI)

in der A, B, $R^1$, $R^2$, $R^3$ und $R^4$ die angebene Bedeutung haben, mit Hydrazin cyclisiert oder

d) falls A, B zusammen eine Bindung bilden und $R^2$ eine Methylgruppe bedeutet, ein Dihydropyridazinon der Formel I

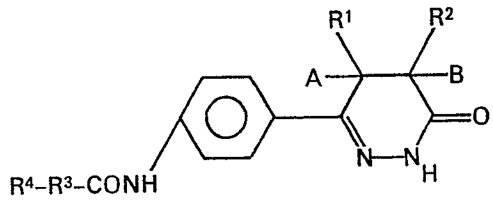

(I),

in der $R^1$, $R^3$ und $R^4$ die oben angegebenen Bedeutungen besitzen und A, B und $R^2$ Wasserstoffatome bedeuten, mit Formaldehyd umsetzt und die so erhaltenen Verbindungen gegebenenfalls in ihre Salze mit physiologisch verträglichen Säuren überführt.

2. Verfahren gemäss Anspruch 1, dadurch gekennzeichnet, dass man 6-(Acylaminoaryl)-3(2H)-pyridazinon-derivate der Formel I gemäss Anspruch 1, in der

A und B Wasserstoffatome sind oder zusammen eine Bindung bilden,

$R^1$ ein Wasserstoffatom oder eine Methylgruppe bedeutet,

$R^2$ ein Wasserstoffatom ist, oder - falls A und B Wasserstoffatome sind, zusammen eine $C_{1-2}$-Alkylengruppe bilden,

$R^3$ eine geradkettige $C_1$-$C_4$-Alkylengruppe, die durch eine $C_1$-$C_3$-Alkylgruppe oder zwei Methylgruppen substituiert sein kann,

$R^4$ a) eine Imidazol-1-yl-Gruppe oder

b) eine Gruppe der Formel II bedeutet, worin $R^5$ ein Wasserstoffatom, eine $C_1$-$C_4$-Alkylgruppe, die gegebenenfalls durch einen Phenylrest substituiert ist, eine $C_3$-$C_6$-Cycloalkylgruppe, eine Phenylgruppe, die gegebenenfalls durch 1 oder 2 $C_1$-$C_4$-Alkyl-, $C_1$-$C_4$-Alkoxygruppen oder Halogenatome und/oder durch eine Nitril-, Nitro- oder Trifluormethylgruppe substituiert sein kann, oder eine Gruppe der Formel $R^7R^8N$ bedeutet, in der $R^7$ ein Wasserstoffatom oder eine Phenylgruppe, die 1- bis 2fach durch Halogenatome, $C_1$-$C_4$-Alkyl-, $C_1$-$C_4$-Alkoxygruppen und/oder eine Nitril- und/oder Nitrogruppe substituiert sein kann, und $R^8$ ein $C_1$-$C_4$-Acyl- oder Benzoylgruppe bedeutet

oder $R^7$ und $R^8$ zusammen mit dem Stickstoffatom eine Benzimidazol-2-on-1-yl-Gruppe bilden, $R^6$ ein Wasserstoffatom, eine $C_1$-$C_4$-Alkyl-, eine Hydroxy-, eine $C_1$-$C_4$-Acyl-, eine ($C_1$-$C_5$-Alkoxy)-carbonyl- oder eine Cyangruppe bedeutet oder $R^5$ und $R^6$ gemeinsam eine $C_1$-$C_4$-Alkylenkette bilden, wobei der gebildete Bicyclus durch 1 bis 3 Methylgruppen substituiert sein kann,
m für 0, 1 oder 2 steht, oder

c) eine Gruppe der Formel III bedeutet, in der $R^9$ einen $C_1$-$C_3$-Kohlenwasserstoffrest, der durch einen Naphthylrest oder einen Phenylrest substituiert ist, wobei der Phenylrest gegebenenfalls 1 bis 2 Halogenatome, $C_1$-$C_4$-Alkyl-, $C_1$-$C_4$-Alkoxygruppen oder eine Trifluormethyl-, Nitro-, Hydroxy-, $C_1$-$C_4$-Acyl-, ($C_1$-$C_5$-Alkoxy)carbonyl- und/oder Cyangruppe enthalten kann, einen Naphthylrest, einen Phenylrest, der 1- bis 3fach durch Halogenatome, $C_1$-$C_4$-Alkyl-, $C_1$-$C_4$-Alkoxygruppen und/oder eine Trifluormethyl- oder eine $C_1$-$C_4$-Acylgruppe substituiert sein kann, einen 6gliedrigen Heteroarylrest mit 1 bis 2 Stickstoffatomen bedeutet,
p die Zahl 2 bedeutet, oder

d) $R^4$ eine Gruppe der Formel IV bedeutet, in der $R^{10}$ einen $C_1$-$C_8$-Alkylrest, der gegebenenfalls durch einen Phenylrest, der 1 bis 2 $C_1$-$C_4$-Alkylgruppen und/oder Halogenatome enthalten kann, substituiert ist, eine $C_3$-$C_{12}$-Cycloalkylgruppe, die durch 1 bis 3 Methylgruppen, einen Phenyl- oder Benzylrest substituiert sein kann, oder einen $C_7$-$C_{10}$bi- oder tricyclischen Alkylrest, der durch 1 bis 3 Methylreste substituiert sein kann, eine benzokondensierte $C_5$-$C_7$-Cycloalkylgruppe bedeutet $R^{11}$ ein Wasserstoffatom oder einen $C_1$-$C_8$-Alkylrest bedeutet, der

e) eine Gruppe der Formel V bedeutet, in der $R^{12}$ und $R^{13}$ Wasserstoff- oder Halogenatome oder $C_{1-4}$-Alkylgruppen bedeuten und n 1 oder 2 ist, oder

f) eine Gruppe der Formel VI bedeutet, in der G ein Sauerstoff- oder Schwefelatom und $R^{14}$ und $R^{15}$ Wasserstoffatome oder Methylgruppen darstellen,

mit der Massgabe, dass, wenn $R^1$, $R^2$, A und B Wasserstoffatome bedeuten, $R^3$ keine, gegebenenfalls durch eine $C_1$-$C_3$-Alkylgruppe substituierte Methylengruppe bedeutet und wenn $R^2$, A und B Wasserstoffatome, $R^1$ ein Wasserstoffatom oder eine Methylgruppe und $R^3$ eine gegebenenfalls durch eine $C_1$-$C_3$-Alkylgruppe substituierte Methylengruppe bedeutet, $R^4$ nicht eine Gruppe der Formel IV darstellen soll, in der $R^{10}$ und $R^{11}$ Wasserstoffatome oder unsubstituierte $C_1$-$C_4$-Alkylgruppen bedeuten, sowie deren Salze mit physiologisch verträglichen Säuren, herstellt.

**Claims for Contracting states: BE, CH, DE, FR, GB, IT, LI, NL, SE**

1. A 6-aryl-4,5-dihydro-3(2H)-pyridazinone derivative of the formula I

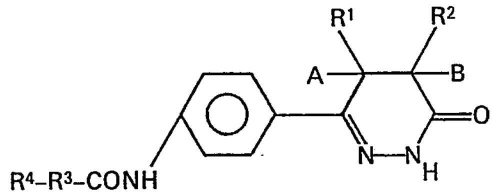

(I),

where
A and B are each hydrogen or together form a bond,
$R^1$ and $R^2$ are each hydrogen or $C_1$-$C_3$-alkyl, or, where A and B are each hydrogen, $R^1$ and $R^2$ together form a $C_1$-$C_4$-alkylene radical, $R^3$ is straight-chain $C_1$-$C_4$-alkylene which may be substituted by 1 or 2 $C_1$-$C_5$-alkyl groups and $R_4$ is

a) pyrrol-1-yl, imidazol-1-yl, pyrazol-1-yl or 1,2,4-triazol-1-yl, or

b) a group of the formula II

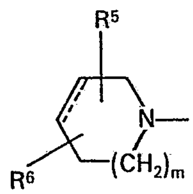

(II)

where the broken line may be an additional bond, $R^5$ and $R^6$ are identical or different and are each hydrogen, a $C_1$-$C_4$-hydrocarbon radical which is unsubstituted or substituted by $C_3$-$C_8$-cycloalkyl or by phenyl which may carry 1, 2 or 3 $C_1$-$C_4$-alkyl, $C_1$-$C_4$-alkoxy, trifluoromethyl and/or nitro groups and/or halogen atoms, or are each $C_3$-$C_8$-cycloalkyl, hydroxyl, trifluoromethyl, $C_1$-$C_4$-acyl, carboxyl, ($C_1$-$C_5$-alkoxy)-carbonyl, cyano, or a $C_6$-$C_{10}$-aryl or 5-membered or 6-membered hetaryl or benzofused hetaryl radical containing 1 to 3 hetero atoms, the aryl and the hetaryl radicals each being unsubstituted or monosubstituted to trisubstituted by halogen, $C_1$-$C_4$-alkyl, $C_1$-$C_4$-alkoxy, hydroxyl, trifluoromethyl, $C_1$-$C_4$-acyl, carboxyl, ($C_1$-$C_5$-alkoxy)carbonyl, cyano, nitro, amino, $C_1$-$C_4$-alkylamino, di-$C_1$-$C_4$-alkylamino, anilino or $C_1$-$C_4$-acylamino, or are each
- a group of the formula $R^7R^8N$-, where $R^7$ and $R^8$ are identical or different and are each hydrogen, $C_1$-$C_8$-alkyl which is unsubstituted or substituted by phenyl which may carry 1, 2, or 3 $C_1$-$C_4$-alkyl, $C_1$-$C_4$-alkoxy, trifluoromethyl and/or nitro groups and/or halogen atoms, or are each phenyl which may be substituted by 1, 2 or 3 halogen atoms or $C_1$-$C_4$-alkyl, $C_1$-$C_4$-alkoxy, trifluoro-methyl, $C_1$-$C_4$-acyl, carboxyl, ($C_1$-$C_5$-alkoxy)carbonyl, cyano and/or nitro groups, or $C_1$-$C_8$-acyl or $C_6$-$C_{10}$-aroyl, where the aroyl group may be monosubstituted to trisubstituted by halogen, $C_1$-$C_4$-alkyl, $C_1$-$C_4$-alkoxy, hydroxyl, trifluoromethyl, $C_1$-$C_4$-alkylthio, $C_1$-$C_4$-alkylsulfoxyl, $C_1$-$C_4$-alkylsulfonyl, $C_1$-$C_4$-acyl, carboxyl, ($C_1$-$C_5$-alkoxy)carbonyl, cyano, nitro, amino, $C_1$-$C_4$-alkylamino, di-$C_1$-$C_4$-alkylamino or $C_1$-$C_4$-acylamino, or the group $R^7R^8N$- is benzimidazol-2-on-1-yl, or
- $R^5$ and $R^6$ together form a $C_1$-$C_4$-alkylene chain,

and the bicyclic structure is unsubstituted or substituted by 1, 2 or 3 $C_1$-$C_3$-alkyl groups, and
- m = 0, 1, 2 or 3, or

c) a group of the formula III

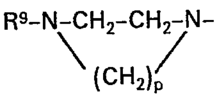

(III)

where $R^9$ is a $C_1$-$C_{14}$-hydrocarbon radical which is unsubstituted or substituted by hydroxyl or $C_3$-$C_8$-cycloalkyl, where $C_5$-$C_8$-cycloalkyl may be benzofused, or by $C_6$-$C_{10}$-aryl which may contain 1, 2 or 3 $C_1$-$C_4$-alkyl, $C_1$-$C_4$-alkoxy, trifluoromethyl, nitro, hydroxyl, $C_1$-$C_4$-acyl, carboxyl, ($C_1$-$C_5$-alkoxy)carbonyl, amido, N-($C_1$-$C_4$-alkyl)amido, N,N-di($C_1$-$C_4$-alkyl)amido, tri-($C_1$-$C_4$-alkyl)silyl, cyano, amino, $C_1$-$C_4$-alkylamino, di($C_1$-$C_4$-alkyl)amino or $C_1$-$C_4$-acylamino groups and/or halogen atoms;

$C_6$-$C_{10}$-aroyl which is unsubstituted or substituted by 1, 2 or 3 $C_1$-$C_4$-alkyl, $C_1$-$C_4$-alkoxy, trifluoromethyl, nitro, hydroxyl, $C_1$-$C_4$-acyl, carboxyl, $C_1$-$C_5$-alkoxycarbonyl, N,N-di($C_1$-$C_4$-alkyl)amido, cyano, di($C_1$-$C_4$-alkyl)amino groups and/or halogen atoms;

hetaroxyl which has 5 or 6 ring members, may contain 1, 2 or 3 hetero atoms and may be benzofused; $C_3$-$C_{12}$-cycloalkyl, $C_1$-$C_8$-acyl,($C_1$-$C_5$-alkoxy)carbonyl, or a $C_6$-$C_{10}$-aryl or 5- or 6-membered hetaryl or benzofused hetaryl radical which contains 1 to 3 nitrogen atoms and may contain an oxygen atom or a sulfur atom, the said aryl or hetaryl radical being unsubstituted or monosubstituted to trisubstituted by halogen, $C_1$-$C_4$-alkyl, $C_1$-$C_4$-alkoxy, hydroxyl, trifluoromethyl, $C_1$-$C_4$-acyl, carboxyl, ($C_1$-$C_5$-alkoxy)-carbonyl, cyano, nitro, amino, $C_1$-$C_4$-alkylamino; di-$C_1$-$C_4$-alkylamino, arylamino or $C_1$-$C_4$-acylamino, and

p is 2 or 3, or

d) a group of the formula IV

$$-NR^{10}R^{11} \quad (IV)$$

where $R^{10}$ and $R^{11}$ are each hydrogen or a $C_1$-$C_{12}$-hydrocarbon radical which is unsubstituted or substituted by one or two phenyl radicals which may carry 1, 2 or 3 $C_1$-$C_4$-alkyl, $C_1$-$C_4$-alkoxy, trifluoromethyl and/or nitro groups and/or halogen atoms, or by naphthyl, $C_3$-$C_8$-cycloalkyl which may contain 1, 2 or 3 $C_1$-$C_4$-alkyl groups, where $C_5$-$C_8$-cycloalkyl may be benzofused, or $C_7$-$C_{10}$-bicycloalkyl which may contain 1, 2 or 3 $C_1$-$C_4$-alkyl groups; $C_3$-$C_{12}$-cycloalkyl which may be substituted by 1, 2 or 3 $C_1$-$C_4$-hydrocarbon radicals which may contain a phenyl which is monosubstituted to trisubstituted by $C_1$-$C_4$-alkyl, $C_1$-$C_4$-alkoxy, trifluoromethyl, nitrile, nitro and/or halogen, or may contain a $C_3$-$C_6$-cycloalkyl group, or by phenyl which may contain 1, 2 or 3 $C_1$-$C_4$-alkyl, $C_1$-$C_4$-alkoxy, trifluoromethyl, nitrile or nitro groups and/or halogen atoms, or by $C_3$-$C_8$-cycloalkyl;

$C_7$-$C_{10}$-bi- or tricyclic alkyl which may be substituted by 1, 2 or 3 $C_1$-$C_4$-alkyl groups, or benzofused $C_5$-$C_7$-cycloalkyl, or

e) a group of the formula V

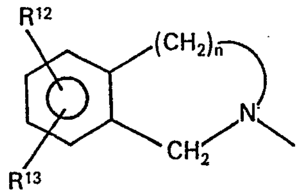

(V)

where $R^{12}$ and $R^{13}$ are each hydrogen, halogen, $C_1$-$C_4$-alkyl or $C_1$-$C_4$-alkoxy and n is 1, 2 or 3, or

f) a group of the formula VI

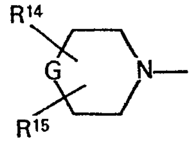

(VI)

where G is oxygen or sulfur and $R^{14}$ and $R^{15}$ are each hydrogen or $C_1$-$C_4$-alkyl,

with the proviso that

when $R^1$, $R^2$, A and B are each hydrogen and the acylamino group is in the para position in the phenyl ring in I, $R^3$ should not be an unsubstituted or $C_1$-$C_5$-alkyl-substituted methylene group, and when $R^2$, A and B are each hydrogen, $R^1$ is hydrogen or $C_1$-$C_3$-alkyl and $R^3$ is an unsubstituted or $C_1$-$C_4$-alkyl-substituted methylene group, $R^4$ should not be a group of the formula IV where $R^{10}$ and $R^{11}$ are each hydrogen or unsubstituted $C_1$-$C_4$-alkyl,

and its salts with physiologically tolerated acids.

2. A 6-(acylaminoaryl)-3(2H)-pyridazinone derivative of the formula I as claimed in claim 1, where A and B are each hydrogen or together form a bond, $R^1$ and $R^2$ are each hydrogen or methyl, or, where A and B are each hydrogen, $R^1$ and $R^2$ together form a $C_1$- or $C_2$-alkylene group, $R^3$ is straight-chain $C_1$-$C_4$-alkylene, which may be substituted by a $C_1$-$C_3$-alkyl group or two methyl groups,

$R^4$ is

a) imidazol-1-yl or

b) a group of the formula II, where D and E have the stated meaning, $R^5$ is hydrogen or $C_1$-$C_4$-alkyl which is unsubstituted or substituted by phenyl, or is $C_3$-$C_6$-cycloalkyl or is phenyl which may be substituted by 1 or 2 $C_1$-$C_4$-alkyl or $C_1$-$C_4$-alkoxy groups or halogen atoms and/or by a nitrile, nitro or trifluoromethyl group, or is a group of the formula $R^7R^8N$, where $R^7$ is hydrogen or phenyl which may be monosubstituted or disubstituted by halogen, $C_1$-$C_4$alkyl, $C_1$-$C_4$-alkoxy and/or nitrile and/or nitro, and $R^8$ is $C_1$-$C_4$-acyl or benzoyl, or $R^7$ and $R^8$ together with the nitrogen atom form a benzimidazol-2-on-1-yl group, $R^6$ is hydrogen, $C_1$-$C_4$-alkyl, hydroxyl, $C_1$-$C_4$-acyl,($C_1$-$C_5$-alkoxy)-carbonyl or cyano, or $R^5$ and $R^6$ together form a $C_1$-$C_4$-alkylene chain, where the resulting bicyclic structure may be substituted by 1, 2 or 3 methyl groups, and

m is 0, 1 or 2, or

c) a group of the formula III, where

$R^9$ is a $C_1$-$C_3$-hydrocarbon radical which is substituted by naphthyl or phenyl, and the phenyl radical may contain 1 or 2 halogen atoms or $C_1$-$C_4$-alkyl or $C_1$-$C_4$-alkoxy groups or a trifluoromethyl, nitro, hydroxyl, $C_1$-$C_4$-acyl, ($C_1$-$C_5$-alkoxy)carbonyl and/or cyano group, or is naphthyl or is phenyl which may be monosubstituted to trisubstituted by halogen, $C_1$-$C_4$-alkyl, $C_1$-$C_4$-alkoxy and/or trifluoromethyl or $C_1$-$C_4$-acyl, or is a 6-membered hetaryl radical containing 1 or 2 nitrogen atoms, and P (sic) is 2, or

d) $R^4$ is a group of the formula IV, where $R^{10}$ is $C_1$-$C_8$-alkyl which is unsubstituted or substituted by phenyl which may contain 1 or 2 $C_1$-$C_4$-alkyl groups and/or halogen atoms, or is $C_3$-$C_{12}$-cycloalkyl which may be substituted by 1, 2 or 3 methyl groups or by a phenyl or benzyl radical, or is a $C_7$-$C_{10}$-bi- or tricyclic alkyl radical which may be substituted by 1, 2 or 3 methyl radicals, or is a benzofused $C_5$-$C_7$-cycloalkyl group, and $R^{11}$ is hydrogen or $C_1$-$C_8$-alkyl, which (sic)

e) a group of the formula V, where $R^{12}$ and $R^{13}$ are each hydrogen, halogen or $C_1$-$C_4$-alkyl and n is 1 or 2, or

f) a group of the formula VI, where G is oxygen or sulfur and $R^{14}$ and $R^{15}$ are each hydrogen or methyl, with the proviso that, when $R^1$, $R^2$, A and B are each hydrogen, $R^3$ is not unsubstituted or $C_1$-$C_3$-alkyl-substituted methylene, and when $R^2$, A and B are each hydrogen, $R^1$ is hydrogen or methyl and $R^3$ is unsubstituted or $C_1$-$C_3$-alkyl-substituted methylene, $R^4$ should not be a group of the formula IV where $R^{10}$ and $R^{11}$ are each hydrogen or unsubstituted $C_1$-$C_4$-alkyl, and its salts with physiologically tolerated acids.

3. 6-{p-[3-(4-phenyl-1-piperazino)-propionylamino]-phenyl}-5-methyl-4,5-dihydro-3(2H)-pyridazinone.

4. 6-{p-[3-(4-pyrid-2-yl-1-piperazino)-propionyl-amino]-phenyl}-5-methyl-4,5-dihydro-3(2H)-pyridazinone.

5. 6-{p-[3-(4-benzyl-1-piperazino)-propionylamino]-phenyl}-5-methyl-4,5-dihydro-3(2H)-pyridazinone.

6. 6-{p-[2-(4-phenyl-1-piperidino)-acetylamino]-phenyl}-5-methyl-4,5-dihydro-3(2H)-pyridazinone.

7. 6-{p-[3-(4-phenyl-1-piperidino)-propionylamino]-phenyl}-4,5-dihydropyridazinone.

8. 6-{p-[3-(4-phenyl-1-piperidino)-propionylamino]-phenyl}-5-methyl-4,5-dihydropyridazinone.

9. 2-{p-[3-(4-phenyl-1-piperidino)-propionylamino]-phenyl}-3,4-diazabicyclo[4.1.0]hept-2-en-5-one.

10. 6-{p-[3-(4-phenyl-1-(1,2,3,6-tetrahydropyridino-propionylamino-]-phenyl}-5-methyl-4,5-dihydro-3-(2H)-pyridazinone.

11. 6-{p-[3-(4-phenyl-1-piperidino)-propionylamino]-phenyl}-3(2H)-pyridazinone.

12. 6-{p-[3-(1-(1,2,3,4-tetrahydroisoquinolino)-propionylamino]-phenyl}-5-methyl-4,5-dihydro-3(2H)-pyridazinone.

13. A process for the preparation of a compound of the formula I as claimed in claim 1, wherein

a) a compound of the formula VII

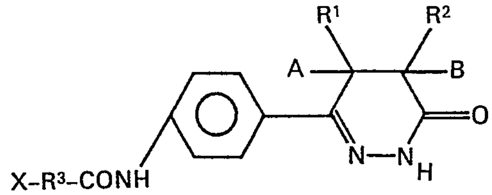

(VII)

where X is halogen and A, B, $R^1$, $R^2$ and $R^3$ have the stated meanings, is reacted with a compound of the formula VIII

$R^4$ - H (VIII)

where $R^4$ has the stated meanings, or

b) an aminophenylpyridazinone of the formula IX

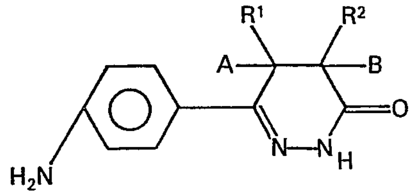

(IX)

where A, B, $R^1$ and $R^2$ have the stated meanings, is acylated in a conventional manner with a compound of the formula X

$R^4$ - $R^3$ - CO - Y . HY (X)

where $R^3$ and $R^4$ have the stated meanings and Y is chlorine or bromine, or

c) ketocarboxylic acid of the formula XI

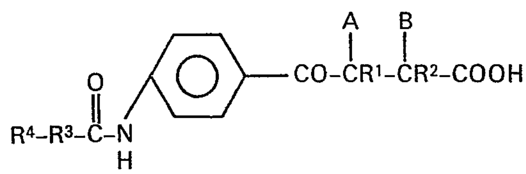

(XI)

where A, B, $R^1$, $R^2$, $R^3$ and $R^4$ have the stated meanings is cyclized with hydrazine, or

d) where A and B together form a bond and $R^2$ is methyl, a dihydropyridazinone of the formula I

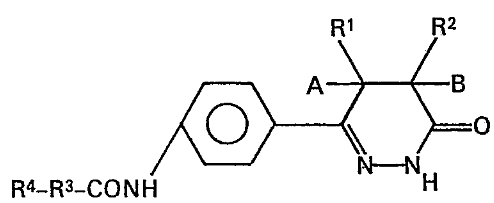

(I)

where $R^1$, $R^3$ and $R^4$ have the abovementioned meanings and A, B and $R^2$ are each hydrogen, is reacted with formaldehyde,
and, if required, the resulting compound is converted to its salt with physiologically tolerated acids.

14. A 6-(acylaminoaryl)-3(2H)-pyridazinone derivative of the formula I as claimed in claim 1 for the treatment of disorders.

**Claims for contracting state: AT**

1. A process for the preparation of a 6-aryl-4,5-dihydro-3(2H)-pyridazinone derivative of the formula I

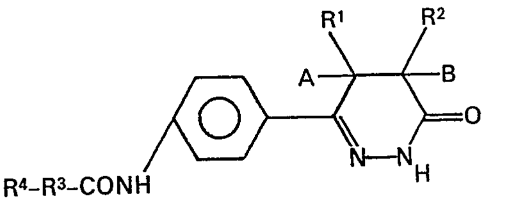

(I),

where
A and B are each hydrogen or together form a bond,
$R^1$ and $R^2$ are each hydrogen or $C_1$-$C_3$-alkyl, or, where A and B are each hydrogen, $R^1$ and $R^2$ together form a $C_1$-$C_4$-alkylene radical, $R^3$ is straight-chain $C_1$-$C_4$-alkylene which may be substituted by 1 or 2 $C_1$-$C_5$-alkyl groups and $R^4$ is

a) pyrrol-1-yl, imidazol-1-yl, pyrazol-1-yl or 1,2,4-triazol-1-yl, or

b) a group of the formula II

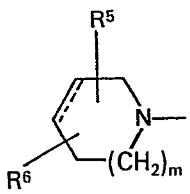

(II)

where the broken line may be an additional bond, $R^5$ and $R^6$ are identical or different and are each hydrogen, a $C_1$-$C_4$-hydrocarbon radical which is unsubstituted or substituted by $C_3$-$C_8$-cycloalkyl or by phenyl which may carry 1, 2 or 3 $C_1$-$C_4$-alkyl, $C_1$-$C_4$-alkoxy, trifluoromethyl and/or nitro groups and/or halogen atoms, or are each $C_3$-$C_8$-cycloalkyl, hydroxyl, trifluoromethyl, $C_1$-$C_4$-acyl, carboxyl, ($C_1$-$C_5$-alkoxy)-carbonyl, cyano, or a $C_6$-$C_{10}$-aryl or 5-membered or 6-membered hetaryl or benzofused hetaryl radical containing 1 to 3 hetero atoms, the aryl and the hetaryl radicals each being unsubstituted or monosubstituted to trisubstituted by halogen, $C_1$-$C_4$-alkyl, $C_1$-$C_4$-alkoxy, hydroxyl, trifluoromethyl, $C_1$-$C_4$-acyl, carboxyl, ($C_1$-$C_5$-alkoxy)carbonyl, cyano, nitro, amino, $C_1$-$C_4$-alkylamino, di-$C_1$-$C_4$-alkylamino, anilino or $C_1$-$C_4$-acylamino, or are each a group of the formula $R^7R^8N$-, where $R^7$ and $R^8$ are identical or different and are each hydrogen, $C_1$-$C_8$-alkyl which is unsubstituted or substituted by phenyl which may carry 1, 2, or 3 $C_1$-$C_4$-aklkyl, $C_1$-$C_4$-alkoxy, trifluoromethyl and/or nitro groups and/or halogen atoms, or are each phenyl which may be substituted by 1, 2 or 3 halogen atoms or $C_1$-$C_4$-alkyl, $C_1$-$C_4$-alkoxy, trifluoro-methyl, $C_1$-$C_4$-acyl, carboxyl, ($C_1$-$C_5$-alkoxy)carbonyl, cyano and/or nitro groups, or $C_1$-$C_8$-acyl or $C_6$-$C_{10}$-aroyl, where the aroyl group may be monosubstituted to trisubstituted by halogen, $C_1$-$C_4$-alkyl, $C_1$-$C_4$-alkoxy, hydroxyl, trifluoromethyl, $C_1$-$C_4$-alkylthio, $C_1$-$C_4$-alkylsulfoxyl, $C_1$-$C_4$-alkylsulfonyl, $C_1$-$C_4$-acyl, carboxyl, ($C_1$-$C_5$-alkoxy)carbonyl, cyano, nitro, amino, $C_1$-$C_4$-alkylamino, di-$C_1$-$C_4$-alkylamino or $C_1$-$C_4$-acylamino, or the group $R^7R^8N$- is benzimidazol-2-on-1-yl, or $R^5$ and $R^6$ together form a $C_1$-$C_4$-alkylene chain, and the bicyclic structure is unsubstituted or substituted by 1, 2 or 3 $C_1$-$C_3$-alkyl groups, and m = 0, 1, 2 or 3, or

c) a group of the formula III

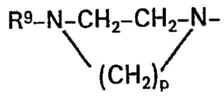

(III)

where $R^9$ is a $C_1$-$C_{14}$-hydrocarbon radical which is unsubstituted or substituted by hydroxyl or $C_3$-$C_8$-cycloalkyl, where $C_5$-$C_8$-cycloalkyl may be benzofused, or by $C_6$-$C_{10}$-aryl which may contain 1, 2 or 3 $C_1$-$C_4$-alkyl, $C_1$-$C_4$-alkoxy, trifluoromethyl, nitro, hydroxyl, $C_1$-$C_4$-acyl, carboxyl, ($C_1$-$C_5$-alkoxy)carbonyl, amido, N-($C_1$-$C_4$-alkyl)amido, N,N-di($C_1$-$C_4$-alkyl)amido, tri($C_1$-$C_4$-alkyl)silyl, cyano, amino, $C_1$-$C_4$-alkylamino, di($C_1$-$C_4$-alkyl)amino or$C_1$-$C_4$-acylamino groups and/or halogen atoms; $C_6$-$C_{10}$-aroyl which is unsubstituted or substituted by 1, 2 or 3 $C_1$-$C_4$-alkyl, $C_1$-$C_4$-alkoxy, trifluoromethyl, nitro, hydroxyl, $C_1$-$C_4$-acyl, carboxyl, $C_1$-$C_5$-alkoxycarbonyl, N,N-di($C_1$-$C_4$-alkyl)-amido, cyano, di-($C_1$-$C_4$-alkyl)amino groups and/or halogen atoms; hetaroyl which has 5 or 6 ring members, may contain 1, 2 or 3 hetero atoms and may be benzofused; $C_3$-$C_{12}$-cycloalkyl, $C_1$-$C_8$-acyl,($C_1$-$C_5$-alkoxy)-carbonyl, or a $C_6$-$C_{10}$-aryl or 5- or 6-membered hetaryl or benzofused hetaryl radical which contains 1 to 3 nitrogen atoms and may contain an oxygen atom or a sulfur atom, the said aryl or hetaryl radical being unsubstituted or monosubstituted to trisubstituted by halogen, $C_1$-$C_4$-alkyl, $C_1$-$C_4$-alkoxy, hydroxyl, trifluoromethyl, $C_1$-$C_4$-acyl, carboxyl, ($C_1$-$C_5$-alkoxy)-carbonyl, cyano, nitro, amino, $C_1$-$C_4$-alkylamino, di-$C_1$-$C_4$-alkylamino, arylamino or $C_1$-$C_4$-acylamino, and
p is 2 or 3, or

d) a group of the formula IV

$$-NR^{10}R^{11} \qquad (IV)$$

where $R^{10}$ and $R^{11}$ are each hydrogen or a $C_1$-$C_{12}$-hydrocarbon radical which is unsubstituted or substituted by one or two phenyl radicals which may carry 1, 2 or 3 $C_1$-$C_4$-alkyl, $C_1$-$C_4$-alkoxy, trifluoromethyl and/or nitro groups and/or halogen atoms, or by naphthyl, $C_3$-$C_8$-cycloalkyl which may contain 1, 2 or 3 $C_1$-$C_4$-alkyl groups, where $C_5$-$C_8$-cycloalkyl may be benzofused, or $C_7$-$C_{10}$-bicycloalkyl which may contain 1, 2 or 3 $C_1$-$C_4$-alkyl groups;
$C_3$-$C_{12}$-cycloalkyl which may be substituted by 1, 2 or 3 $C_1$-$C_4$-hydrocarbon radicals which may contain a phenyl which is monosubstituted to trisubstituted by $C_1$-$C_4$-alkyl, $C_1$-$C_4$-alkoxy, trifluoromethyl, nitrile, nitro and/or halogen, or may contain a $C_3$-$C_6$-cycloalkyl group, or by phenyl which may contain 1, 2 or 3 $C_1$-$C_4$-alkyl, $C_1$-$C_4$-alkoxy, trifluoromethyl, nitrile or nitro groups and/or halogen atoms, or by $C_3$-$C_8$-cycloalkyl;

$C_7$-$C_{10}$-bi- or tricyclic alkyl which may be substituted by 1, 2 or 3 $C_1$-$C_4$-alkyl groups, or benzofused $C_5$-$C_7$-cycloalkyl, or

e) a group of the formula V

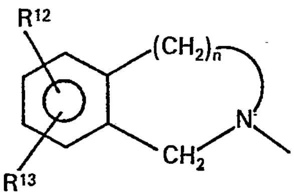

(V)

where $R^{12}$ and $R^{13}$ are each hydrogen, halogen, $C_1$-$C_4$-alkyl or $C_1$-$C_4$-alkoxy and n is 1, 2 or 3, or

f) a group of the formula VI

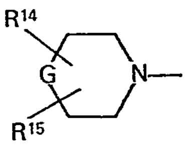

(VI)

where G is oxygen or sulfur and $R^{14}$ and $R^{15}$ are each hydrogen or $C_1$-$C_4$-alkyl,
with the proviso that
when $R^1$, $R^2$, A and B are each hydrogen and the acylamino group is in the para position in the phenyl ring in I, $R^3$ should not be an unsubstituted or $C_1$-$C_5$-alkyl-substituted methylene group, and when $R^2$, A and B are each hydrogen, $R^1$ is hydrogen or $C_1$-$C_3$-alkyl and $R^3$ is an unsubstituted or $C_1$-$C_4$-alkyl-substituted methylene group, $R^4$ should not be a group of the formula IV where $R^{10}$ and $R^{11}$ are each hydrogen or unsubstituted $C_1$-$C_4$-alkyl,
and its salts with physiologically tolerated acids, wherein

a) a compound of the formula VII

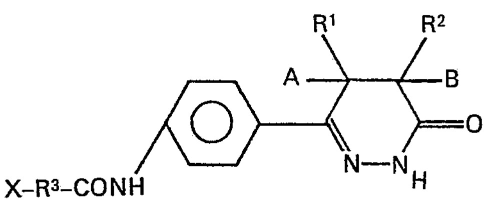

(VII)

where X is halogen and
A, B, $R^1$, $R^2$ and $R^3$ have the stated meanings, is reacted with a compound of the formula VIII

$R^4$ - H (VIII)

where $R^4$ has the stated meanings, or

b) an aminophenylpyridazinone of the formula IX

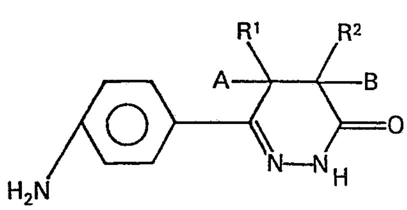

(IX)

where A, B, $R^1$ and $R^2$ have the stated meanings, is acylated in a conventional manner with a compound of the formula X

$R^4$ - $R^3$ - CO - Y. HY (X)

where $R^3$ and $R^4$ have the stated meanings and Y is chlorine or bromine, or

c) a ketocarboxylic acid of the formula XI

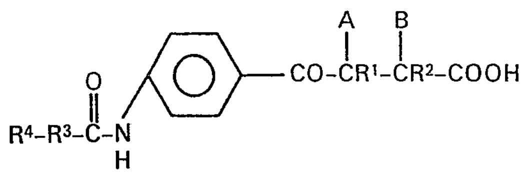

(XI)

where A, B, $R^1$, $R^2$ $R^3$ and $R^4$ have the stated meanings is cyclized with hydrazine, or

d) where A and B together form a bond and $R^2$ is methyl, a dihydropyridazinone of the formula I

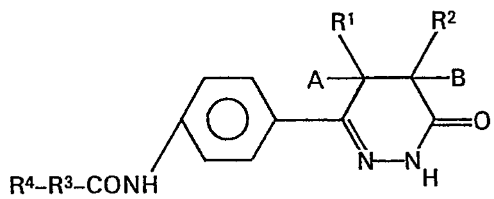

(I)

where $R^1$, $R^3$ and $R^4$ have the abovementioned meanings and A, B and $R^2$ are each hydrogen, is reacted with formaldehyde,
and, if required, the resulting compound is converted to its salts with physiologically tolerated acids.

2. Process as claimed in claim 1, wherein a 6-(acylaminoaryl)-3(2H)-pyridazinone derivative of the formula I as claimed in claim 1, where A and B are each hydrogen or together form a bond,
$R^1$ and $R^2$ are each hydrogen or methyl, or, where A and B are each hydrogen, $R^1$ and $R^2$ together form a $C_1$- or $C_2$-alkylene group, $R^3$ is straight-chain $C_1$-$C_4$-alkylene, which may be substituted by a $C_1$-$C_3$-alkyl group or two methyl groups,
$R^4$ is

a) imidazol-1-yl or

b) a group of the formula II, where D and E have the stated meaning, $R^5$ is hydrogen or $C_1$-$C_4$-alkyl which is unsubstituted or substituted by phenyl, or is $C_3$-$C_6$-cycloalkyl or is phenyl which may be substituted by 1 or 2 $C_1$-$C_4$-alkyl or $C_1$-$C_4$-alkoxy groups or halogenatoms and/or by a nitrile, nitro or trifluoromethyl group,
or is a group of the formula $R^7R^8N$, where $R^7$ is hydrogen or phenyl which may be monosubstituted or disubstituted by halogen, $C_1$-$C_4$-alkyl, $C_1$-$C_4$-alkoxy and/or nitrile and/or nitro, and $R^8$ is $C_1$-$C_4$-acyl or benzoyl, or $R^7$ and $R^8$ together with the nitrogen atom form a benzimidazol-2-on-1-yl group, $R^6$ is hydrogen, $C_1$-$C_4$-alkyl, hydroxyl, $C_1$-$C_4$-acyl, ($C_1$-$C_5$-alkoxy)-carbonyl or cyano, or $R^5$ and $R^6$ together form a $C_1$-$C_4$-alkylene chain, where the resulting bicyclic structure may be substituted by 1, 2 or 3 methyl groups, and
m is 0, 1 or 2, or

c) a group of the formula III, where $R^9$ is a $C_1$-$C_3$-hydrocarbon radical which is substituted by naphthyl or phenyl, and the phenyl radical may contain 1 or 2 halogen atoms or $C_1$-$C_4$-alkyl or $C_1$-$C_4$-alkoxy groups or a trifluoromethyl, nitro, hydroxyl, $C_1$-$C_4$-acyl, ($C_1$-$C_5$-alkoxy)-carbonyl and/or cyano group, or is naphthyl or is phenyl which may be monosubstituted to trisubstituted by halogen, $C_1$-$C_4$-alkyl, $C_1$-$C_4$-alkoxy and/or trifluoromethyl or $C_1$-$C_4$-acyl, or is a 6-membered hetaryl radical containing 1 or 2 nitrogen atoms, and p (sic) is 2,or

d) $R^4$ is a group of the formula IV, where $R^{10}$ is $C_1$-$C_8$-alkyl which is unsubstituted or substituted by phenyl which may contain 1 or 2 $C_1$-$C_4$-alkyl groups and/or halogen atoms, or is $C_3$-$C_{12}$-cycloalkyl which may be substituted by 1, 2 or 3 methyl groups or by a phenyl or benzyl radical, or is a $C_7$-$C_{10}$-bi- or tricyclic alkyl radical which may be substituted by 1, 2 or 3 methyl radicals, or is a benzofused $C_5$-$C_7$-cycloalkyl group, and $R^{11}$ is hydrogen or $C_1$-$C_8$-alkyl, which (sic)

e) a group of the formula V, where $R^{12}$ and $R^{13}$ are each hydrogen, halogen or $C_1$-$C_4$-alkyl and n is 1 or 2, or

f) a group of the formula VI, where G is oxygen or sulfur and $R^{14}$ and $R^{15}$ are each hydrogen or methyl,
with the proviso that, when $R^1$, $R^2$, A and B are each hydrogen, $R^3$ is not unsubstituted or $C_1$-$C_3$-alkyl-substituted methylene, and when $R^2$, A and B are each hydrogen, $R^1$ is hydrogen or methyl and $R^3$ is unsubstituted or $C_1$-$C_3$-alkyl-substituted methylene, $R^4$ should not be a group of the formula IV where $R^{10}$ and $R^{11}$ are each hydrogen or unsubstituted $C_1$-$C_4$-alkyl, and its salts with physiologically tolerated acids, is prepared.

**Revendications pour les Etats Contrac tants: BE, CH, DE, FR, GB, IT, LI, NL, SE**

1. Dérivés de 6-aryl-4,5-dihydro-3(2H)-pyridazinones de la formule I

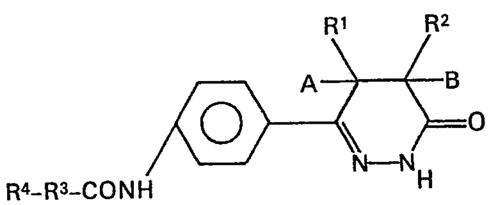
(I),

dans laquelle
A et B représentent des atomes d'hydrogène ou forment ensemble une liaison,
$R^1$ désigne un atome d'hydrogène ou un radical alkyle en $C^1$ à $C_3$ et
$R^2$ désigne un atome d'hydrogène ou, lorsque A=B=H,
$R^1$ et $R^2$ représentent ensemble un groupe alkylène en $C_1$ à $C_4$,
$R^3$désigne un groupe alkylène à chaîne droite en $C_1$ à $C_4$, pouvant être substitué par un ou deux radicaux alkyle en $C_1$ à $C_5$ et
$R^4$ représente

a) un groupe pyrrole-1-yle, imidazole-1-yle, pyrazole-1-yle ou 1,2,4-triazole-1-yle;

b) un groupe de la formule II

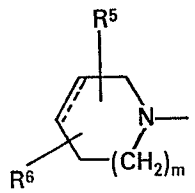
(II),

dans laquelle
la ligne interrompue peut représenter une deuxième liaison,
$R^5$ et $R^6$, qui peuvent être identiques ou différents, représentent des atomes d'hydrogène, des groupes hydrocarbonés en $C_1$ à $C_4$, éventuellement substitués par un groupe cycloalkyle en $C_3$ à $C_8$ ou par un groupe phényle, pouvant lui-même être substitué par un à trois radicaux alkyle en $C_1$ à $C_4$ ou alcoxy en $C_1$ à $C_4$, groupes trifluorométhyle et(ou) nitro et(ou) atomes d'halogène, des groupes cycloalkyle en $C_3$ à $C_8$, hydroxyle, trifluorométhyle, acyle en $C_1$ à $C_4$, carboxyle, alcoxy (en $C_1$ à $C_5$)-carbonyle ou cyano ou des groupes aryle en $C_6$ à $C_{10}$ ou hétéro-aryle penta- ou hexagonaux avec un à trois hétéro-atomes, éventuellement benzoconjugués et pouvant porter un à trois substituants choisis parmi les atomes d'halogène et les groupes alkyle en $C_1$ à $C_4$, alcoxy en $C_1$ à $C_4$, hydroxyle, trifluorométhyle, acyle en $C_1$ à $C_4$, carboxyle, alcoxy (en $C_1$ à $C_5$)-carbonyle, cyano, nitro, amino, alkyl (en $C_1$ à $C_4$)-amino, dialkyl (en $C_1$ à $C_4$)-amino, anilino et acyl (en $C_1$ à $C_4$)-amino, ou des groupes de la formule $R^7R^8N$-, dans laquelle $R^7$ et $R^8$, qui peuvent être identiques ou différents, désignent des atomes d'hydrogène, des radicaux alkyle en $C_1$ à $C_8$ éventuellement substitués par un groupe phényle pouvant porter un à trois substituants choisis parmi les groupes alkyle en $C_1$ à $C_4$, alcoxy en $C_1$ à $C_4$, trifluoro-méthyle et(ou) nitro et (ou) les atomes d'halogène, des groupes phényle pouvant être substitués par un à trois atomes d'halogène ou groupes alkyle en $C_1$ à $C_4$, alcoxy en $C_1$ à $C_4$, trifluorométhyle, acyle en $C_1$ à $C_4$, carboxyle, alcoxy (en $C_1$ à $C_5$)-carbonyle, cyano et(ou) nitro, ou des groupes acyle en $C_1$ à $C_8$ ou aroyle en $C_6$ à $C_{10}$, les groupes aroyle pouvant porter un à trois substituants choisis parmi les atomes d'halogène et les groupes alkyle en $C_1$ à $C_4$, alcoxy en $C_1$ à $C_4$, hydroxyle, trifluorométhyle, alkyl(en $C_1$ à $C_4$)-thio, alkyl (en $C_1$ à $C_4$)-sulfoxyle, alkyl(en $C_1$ à $C_4$)-sulfonyle, acyle en $C_1$ à $C_4$, carboxyle, alcoxy (en $C_1$ à $C_5$)-carbonyle, cyano, nitro, amino, alkyl (en $C_1$ à $C_4$)-amino, dialkyl (en $C_1$ à $C_4$)-amino et acyl (en $C_1$ à $C_4$)-amino, le groupe $R^7R^8N$-pouvant aussi désigner le reste benzimidazole-2-one-1-yle,
$R^5$ et $R^6$ pouvant en outre former ensemble un pont alkylène en $C_1$ à $C_4$, le composé bicyclique pouvant éventuellement être substitué par un à trois radicaux alkyle en $C_1$ à $C_3$, et
n vaut 0, 1, 2 ou 3;

c) un groupe de la formule III

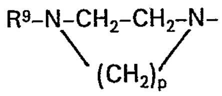

(III),

dans laquelle

$R^9$ représente un groupe hydrocarboné en $C_1$ à $C_{14}$ éventuellement substitué par un groupe hydroxyle, un groupe cycloalkyle en $C_3$ à $C_8$ avec éventuellement un groupe cycloalkyle en $C_5$ à $C_8$ benzo-conjugué ou un groupe aryle en $C_6$ à $C_{10}$, pouvant porter un à trois substituants choisis parmi les radicaux alkyle en $C_1$ à $C_4$ et alcoxy en $C_1$ à $C_4$, les groupes trifluorométhyle, nitro, hydroxyle, acyle en $C_1$ à $C_4$, carboxyle, alcoxy (en $C_1$ à $C_5$)-carbonyle, amido, N-alkyl (en $C_1$ à $C_4$)-amido, N,N-dialkyl en $C_1$ à $C_4$-amido, tri-alkyl (en $C_1$ à $C_4$)-silyle, cyano, amino, alkyl (en $C_1$ à $C_4$)-amino, di-alkyl (en $C_1$ à $C_4$)-amino, acyl (en $C_1$ à $C_4$)-amino et(ou) les atomes d'halogène;
un groupe aroyle en $C_6$ à $C_{10}$ portant éventuellement un à trois substituants choisis parmi les radicaux alkyle en $C_1$ à $C_4$ et alcoxy en $C_1$ à $C_4$, les groupes trifluorométhyle, nitro, hydroxyle, acyle en $C_1$ à $C_4$, carboxyle, alcoxy (en $C_1$ à $C_5$)-carbonyle, N,N-di-alkyl (en $C_1$ à $C_4$)-amido, cyano et di-alkyl (en $C_1$ à $C_4$)-amino et(ou) les atomes d'halogène;
un groupe aroyle hétérocyclique penta- ou hexagonal avec un à trois hétéro-atomes, éventuellement benzo-conjugué, un groupe cycloalkyle en $C_3$ à $C_{12}$, acyle en $C_1$ à $C_8$ ou alcoxy (en $C_1$ à $C_5$)-carbonyle, un groupe aryle en $C_6$ à $C_{10}$ pouvant porter un à trois substituants choisis parmi les atomes d'halogène, les radicaux alkyle en $C_1$ à $C_4$ et alcoxy en $C_1$ à $C_4$ et les groupes hydroxyle, trifluorométhyle, acyle en $C_1$ à $C_4$, carboxyle, alcoxy (en $C_1$ à $C_5$)-carbonyle, cyano, nitro, amino, alkyl (en $C_1$ à $C_4$)-amino, di-alkyl (en $C_1$ à $C_4$)-amino, aryl-amino et acyl (en $C_1$ à $C_4$)-amino, ou un groupe aryle hétérocyclique penta- ou hexagonal, éventuellement benzo-conjugué, qui comprend un à trois atomes d'azote et le cas échéant un atome d'oxygène ou un atome de soufre, et
p vaut 2 ou 3;

d) un groupe de la formule IV

$-NR^{10}R^{11}$ (IV),

dans laquelle $R^{10}$ et $R^{11}$ désignent chacun un atome d'hydrogène ou un groupe hydrocarboné en $C_1$ à $C_{12}$, pouvant être substitué par un ou deux groupes phényle, comportant éventuellement un à trois substituants choisis parmi les radicaux alkyle en $C_1$ à $C_4$ et alcoxy en $C_1$ à $C_4$, les groupes trifluorométhyle et(ou) nitro et(ou) les atomes d'halogène, un groupe naphthyle ou cycloalkyle en $C_3$ à $C_8$, comportant un à trois radicaux alkyle en $C_1$ à $C_4$, des groupes cycloalkyle en $C_5$ à $C_8$ pouvant être benzo-conjugués, ou un groupe bicyclo-alkyle en $C_7$ à $C_{10}$, portant un à trois radicaux alkyle en $C_1$ à $C_4$,
un groupe cycloalkyle en $C_3$ à $C_{12}$ portant éventuellement un à trois substituants choisis parmi les groupes hydrocarbonés en $C_1$ à $C_4$, pouvant porter un groupe phényle mono- à tri-substitué par des atomes d'halogène et(ou) des groupes alkyle en $C_1$ à $C_4$, alcoxy en $C_1$ à $C_4$, trifluoro-méthyle, nitrile et nitro, ou un groupe cycloalkyle en $C_3$ à $C_6$, ou un groupe phényle mono- à tri-substitué par des groupes alkyle en $C_1$ à $C_4$, alcoxy en $C_1$ à $C_4$, trifluorométhyle, nitrile ou nitro et(ou) des atomes d'halogène ou par un groupe cycloalkyle en $C_3$ à $C_8$, un groupe alkyle bi- ou tri-cyclique en $C_7$ à $C_{10}$, pouvant être mono-à tri-substitué par des radicaux alkyle en $C_1$ à $C_4$ ou
un groupe cycloalkyle en $C_5$ à $C_7$ benzo-conjugué;

e) un groupe de la formule V

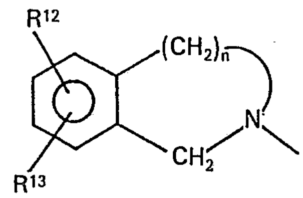

(V),

dans laquelle $R^{12}$ et $R^{13}$ désignent chacun un atome d'hydrogène ou d'halogène ou un radical alkyle en $C_1$ à $C_4$ ou alcoxy en $C_1$ à $C_4$ et n vaut 1, 2 ou 3;

f) un groupe de la formule VI

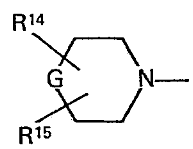

(VI),

dans laquelle G désigne un atome d'oxygène ou de soufre et $R^{14}$ et $R^{15}$ désignent chacun un atome d'hydrogène ou un radical alkyle en $C_1$ à $C_4$, avec les conditions que,
si $R^1$, $R^2$, A et B représentent des atomes d'hydrogène (et le groupe acyl-amino est en position para sur le noyau phénylique dans la formule I), $R^3$ n'est pas un groupe méthylène éventuellement alkyl (en $C_1$ à $C_5$)-substitué, et
si $R^2$, A et B désignent des atomes d'hydrogène, $R^1$=H ou alkyle en $C_1$ à $C_3$ et $R^3$ désigne un pont méthylène éventuellement alkyl (en $C_1$ à $C_4$)- substitué, $R^4$ n'est pas un groupe de la formule IV, dans laquelle $R^{10}$ et $R^{11}$ représentent des atomes d'hydrogène ou des radicaux alkyle en $C_1$ à $C_4$ non substitués,
ainsi que les sels de ces dérivés et d'acides physiologiquement compatibles.

2. Dérivés de 6-(acylaminoaryl)-3(2H)-pyridazinones de la formule I selon la revendication 1, dans laquelle
A et B sont des atomes d'hydrogène ou forment ensemble une liaison,
- $R^1$ est un atome d'hydrogène ou un radical méthyle et
$R^2$ est un atome d'hydrogène ou, lorsque A=B=H, $R^1$ et $R^2$ forment ensemble un groupe alkylène en $C_1$ ou $C_2$,
$R^3$ désigne un groupe alkylène à chaîne droite en $C_1$ à $C_4$, pouvant être substitué par un radical alkyle en $C_1$ à $C_3$ ou deux radicaux méthyle,
$R^4$ désigne

a) un groupe imidazole-1-yle;

b) un groupe de la formule II, dans laquelle $R^5$ désigne un atome d'hydrogène, un radical alkyle en $C_1$ à $C_4$, pouvant être substitué par un groupe phényle, un groupe cycloalkyle en $C_3$ à $C_6$, un groupe phényle pouvant être substitué par un ou deux radicaux alkyle en $C_1$ à $C_4$ ou alcoxy en $C_1$ à $C_4$ ou atomes d'halogène et(ou) par un groupe nitrile, nitro ou trifluorométhyle, ou un groupe de la formule $R^7R^8N$, dans laquelle $R^7$ est un atome d'hydrogène ou un groupe phényle pouvant être substitué par un ou deux atomes d'halogène ou radicaux alkyle en $C_1$ à $C_4$ ou alcoxy en $C_1$ à $C_4$ et (ou) par un groupe nitrile et(ou) nitro et $R^8$ est un groupe acyle en $C_1$ à $C_4$ ou benzoyle ou $R^7$ et $R^8$ forment avec l'atome d'azote adjacent un groupe benzimidazole-2-one-1-yle, et
$R^6$ est un atome d'hydrogène ou un groupe alkyle en $C_1$ à $C_4$, hydroxyle, acyle en $C_1$ à $C_4$, alcoxy (en $C_1$ à $C_5$)-carbonyle ou cyano ou
$R^5$ et $R^6$ forment ensemble un pont alkylène en $C_1$ à $C_4$, le composé bicyclique formé pouvant être substitué par un à trois radicaux méthyle,
vaut 0, 1 ou 2;

c) un groupe de la formule III, dans laquelle $R^9$ désigne un groupe hydrocarboné en $C_1$ à $C_3$, substitué par un groupe naphtyle ou phényle, le groupe phényle pouvant porter un ou deux atomes d'halogène, radicaux alkyle en $C_1$ à $C_4$ ou alcoxy en $C_1$ à $C_4$ ou un groupe trifluorométhyle, nitro, hydroxyle, acyle en $C_1$ à $C_4$, alcoxy (en $C_1$ à $C_5$)-carbonyle et(ou) cyano, un groupe naphtyle, un groupe phényle mono- à tri-substitué par des atomes d'halogène ou radicaux alkyle en $C_1$ à $C_4$ ou alcoxy en $C_1$ à $C_4$ et(ou) mono-substitué par un groupe trifluorométhyle ou un groupe acyle en $C_1$ à $C_4$, ou un groupe aryle hexagonal hétérocyclique avec un ou deux atomes d'azote et vaut 2;

d) ($R^4$) représente un groupe de la formule IV, dans laquelle
$R^{10}$ désigne un radical alkyle en $C_1$ à $C_8$, éventuellement substitué par un groupe phényle pouvant porter un ou deux radicaux alkyle en $C_1$ à $C_4$ et(ou) atomes d'halogène, un groupe cycloalkyle en $C_3$ à $C_{12}$ pouvant être substitué par un à trois radicaux méthyle ou un groupe phényle ou benzyle, un groupe alkyle bi- ou tri-cyclique en $C_7$ à $C_{10}$ pouvant être substitué par un à trois radicaux méthyle ou un groupe cycloalkyle en $C_5$ à $C_7$ benzo-conjugué, et
$R^{11}$ est un atome d'hydrogène ou un radical alkyle en $C_1$ à $C_8$;

e) ($R^4$) est un groupe de la formule V, dans laquelle $R^{12}$ et $R^{13}$ désignent chacun un atome d'hydrogène ou d'halogène ou un radical alkyle en $C_1$ à $C_4$ et n vaut 1 ou 2;

f) ($R^4$) est un groupe de la formule VI, dans laquelle G désigne un atome d'oxygène ou de soufre et $R^{14}$ et $R^{15}$ désignent chacun un atome d'hydrogène ou un radical méthyle, avec les conditions que, lorsque $R^1=R^2=A=B=H$, $R^3$ n'est pas un groupe méthylène éventuellement substitué par un radical alkyle en $C_1$ à $C_3$, et lorsque $R^2=A=B=H$, $R^1=H$ ou-$CH_3$ et $R^3$ est un groupe méthylène éventuellement substitué par un radical alkyle en $C_1$ à $C_3$, $R^4$ ne doit pas signifier un groupe de la formule IV, dans laquelle $R^{10}$ et $R^{11}$ sont des atomes d'hydrogène ou des radicaux alkyle en $C_1$ à $C_4$ non substitués,
ainsi que les sels de ces dérivés et d'acides physiologiquement acceptables.

3. La 6-{p-[3-(4-phényl-1-pipérazino)-propionyl-amino]-phényl}-5-méthyl-4,5-dihydro-3(2H)-pyridazinone.

4. La 6-{p-[3-(4-pyrid-2-yl-1-pipérazino)-propionylamino]-phényl}-5-méthyl-4,5-dihydro-3(2H)-pyridazinone.

5. La 6-{p-[3-(4-benzyl-1-pipérazino)-propionyl-amino]-phényl}-5-méthyl-4,5-dihydro-3(2H)-pyridazinone.

6. La 6-{p-[2-(4-phényl-1-pipéridino)-acétyl-amino]-phényl}-5-méthyl-4,5-dihydro-3(2H)-pyridazinone.

7. La 6-{[p-3-(4-phényl-1-pipéridino)-propionyl-amino]-phényl}-4,5-dihydropyridazinone.

8. La 6-{p-[3-(4-phényl-1-pipéridino)-propionyl-amino]-phényl}-5-méthyl-4,5-dihydropyridazinone.

9. La 2-{p-[3-(4-phényl-1-pipéridino)-propionyl-amino]-phényl}-3,4-diazabicyclo[4.1.0]heptène-(2)-one-(5).

10. La 6-{p-[3-(4-phényl-1-(1,2,3,6-tétrahydropyridino)-propionylamino]-phényl}-5-méthyl-4,5-dihydro-3(2H)-pyridazinone.

11. La 6-{p-[3-(4-phényl-1-pipéridino)-propionyl-amino]-phényl}-3-(2H)-pyridazinone.

12. La 6-{p-[3-(1-(1,2,3,4-tétrahydroiso-quinoléino)-propionylamino]-phényl}-5-méthyl-4,5-dihydro-3(2H)pyridazinone.

13. Procédé de préparation de composés de la formule I selon la revendication 1, caractérisé en ce que:

a) on fait réagir un composé de la formule VII

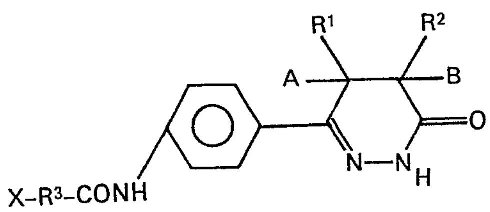
(VII),

dans laquelle A, B, $R^1$, $R^2$ et $R^3$ possèdent les significations définies et X désigne un atome d'halogène, avec un composé de la formule VIII

$R^4$ - H (VIII),

dans laquelle $R^4$ possède les significations définies, ou

b) on soumet une aminophényl-pyridazinone de la formule IX

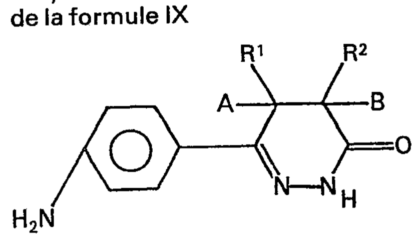
(IX)

dans laquelle A, B, $R^1$ et $R^2$ possèdent les significations définies, à une acylation connue en soi par un composé de la formule X

$R^4-R^3-CO-Y\cdot HY$ (X),

dans laquelle $R^3$ et $R^4$ possèdent les significations définies et Y désigne un atome de chlore ou de brome;

ou

c) on soumet un acide céto-carboxylique de la formule XI

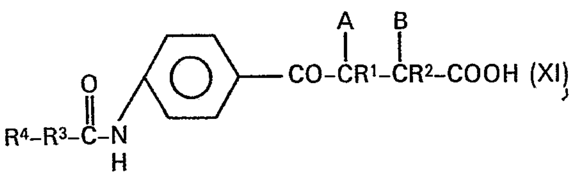

(XI)

dans laquelle A, B, $R^1$, $R^2$, $R^3$ et $R^4$ possèdent la signification définie, à une cyclisation à l'aide d'hydrazine; ou

d) lorsque A et B forment ensemble une liaison et $R^2$ désigne un radical méthyle, on fait réagir une dihydro-pyridazinone de la formule I

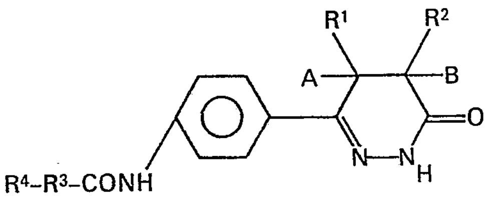

(I),

dans laquelle $R^1$, $R^3$ et $R^4$ possèdent les significations définies précédemment et $A=B=R^2=H$, avec l'aldéhyde formique,

les composés obtenus pouvant, le cas échéant, être transformés en leurs sels d'acides physiologiquement acceptables.

14. Dérivés de 6-(acylaminoaryl)-3(2H)-pyridazinones de la formule I selon la revendication 1, utilisés pour le traitement de maladies (d'états maladifs).

**Revendications pour les Etats contractants: AT**

1. Procédé de préparation de dérivés de 6-aryl-4,5-dihydro-3(2H)-pyridazinones de la formule I

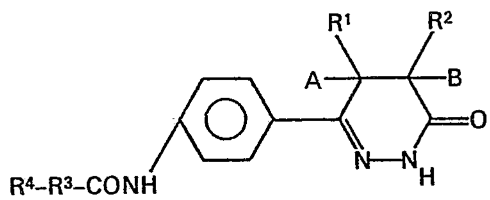

(I),

dans laquelle

A et B représentent des atomes d'hydrogène ou forment ensemble une liaison,

$R^1$ désigne un atome d'hydrogène ou un radical alkyle en $C_1$ à $C_3$ et

$R^2$ désigne un atome d'hydrogène

ou, lorsque A=B=H,

$R^1$ et $R^2$ représentent ensemble un groupe alkylène en $C_1$ à $C_4$,

$R^3$ désigne un groupe alkylène à chaîne droite en $C_1$ à $C_4$, pouvant être substitué par un ou deux radicaux alkyle en $C_1$ à $C_5$ et

$R^4$ représente

a) un groupe pyrrole-1-yle, imidazole-1-yle, pyrazole-1-yle ou 1,2,4-triazole-1-yle;

b) un groupe de la formule II

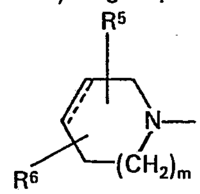

(II)

dans laquelle

la ligne interrompue peut représenter une deuxième liaison,

$R^5$ et $R^6$, qui peuvent être identiques ou différents, représentent des atomes d'hydrogène, des groupes hydrocarbonés en $C_1$ à $C_4$, éventuellement substitués par un groupe cycloalkyle en $C_3$ à $C_8$ ou par un groupe phényle, pouvant lui-même être substitué par un à trois radicaux alkyle en $C_1$ à $C_4$ ou alcoxy en $C_1$ à $C_4$, groupes trifluorométhyle et(ou) nitro et(ou) atomes d'halogène, des groupes cycloalkyle en $C_3$ à $C_8$, hydroxyle, trifluorométhyle, acyle en $C_1$ à $C_4$, carboxyle, alcoxy (en $C_1$ à $C_5$)-carbonyle ou cyano ou des groupes aryle en $C_6$ à $C_{10}$ ou hétéro-aryle penta- ou hexagonaux avec un à trois hétéro-atomes, éventuellement benzoconjugués et pouvant porter un à trois substituants choisis parmi les atomes d'halogène et les groupes alkyle en $C_1$ à $C_4$, alcoxy en $C_1$ à $C_4$, hydroxyle, trifluorométhyle, acyle en $C_1$ à $C_4$, carboxyle, alcoxy (en $C_1$ à $C_5$)-carbonyle, cyano, nitro, amino, alkyl (en $C_1$ à $C_4$)-amino, dialkyl (en $C_1$ à $C_4$)-amino, anilino et acyl (en $C_1$ à $C_4$)-amino, ou des groupes de la formule $R^7R^8N$-, dans laquelle $R^7$ et $R^8$, qui peuvent être identiques ou différents, désignent des atomes d'hydrogène, des radicaux alkyle en $C_1$ à $C_8$ éventuellement substitués par un groupe phényle pouvant porter un à trois substituants choisis parmi les groupes alkyle en $C_1$ à $C_4$, alcoxy en $C_1$ à $C_4$, trifluoro-méthyle et(ou) nitro et(ou) les atomes d'halogène des groupes phényle pouvant être substitués par un a trois atomes d'halogène ou groupes alkyle en $C_1$ à $C_4$, alcoxy en $C_1$ à $C_4$, trifluorométhyle, acyle en $C_1$ à $C_4$, carboxyle, alcoxy (en $C_1$ à $C_5$)-carbonyle, cyano et(ou) nitro, ou des groupes acyle en $C_1$ à $C_8$ ou aroyle en $C_6$ à $C_{10}$, les groupes aroyle pouvant porter un à trois substituants choisis parmi les atomes d'halogène et les groupes alkyle en $C_1$ à $C_4$, alcoxy en $C_1$ à $C_4$, hydroxyle, trifluorométhyle, alkyl (en $C_1$ à $C_4$)-thio, alkyl (en $C_1$ à $C_4$)-sulfoxyle, alkyl (en $C_1$ à $C_4$)-sulfonyle, acyle en $C_1$ à $C_4$, carboxyle, alcoxy (en $C_1$ à $C_5$)-carbonyle, cyano, nitro, amino, alkyl (en $C_1$ à $C_4$)-amino, dialkyl (en $C_1$ à $C_4$)-amino et acyl (en $C_1$ à $C_4$)-amino, le groupe $R^7R^8N$-pouvant aussi désigner le reste benzimidazole-2-one-1-yle,

$R^5$ et $R^6$ pouvant en outre former ensemble un pont alkylène en $C_1$ à $C_4$, le composé bicyclique pouvant éventuellement être substitué par un à trois radicaux alkyle en $C_1$ à $C_3$, et
n vaut 0, 1, 2 ou 3;
c) un groupe de la formule III

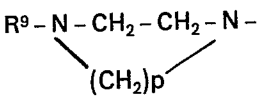
(III),

dans laquelle
$R^9$ représente un groupe hydrocarboné en $C_1$ à $C_{14}$ éventuellement substitué par un groupe hydroxyle, un groupe cycloalkyle en $C_3$ à $C_8$ avec éventuellement un groupe cycloalkyle en $C_5$ à $C_8$ benzo-conjugué ou un groupe aryle en $C^6$ à $C_{10}$, pouvant porter un à trois substituants choisis parmi les radicaux alkyle en $C_1$ à $C_4$ et alcoxy en $C_1$ à $C_4$, les groupes trifluoro-méthyle, nitro, hydroxyle, acyle en $C_1$ à $C_4$, carboxyle, alcoxy (en $C_1$ à $C_5$)-carbonyle, amido, N-alkyl (en $C_1$ à $C_4$)-amido, N,N-dialkyl (en $C_1$ à $C_4$)-amido, tri-alkyl (en $C_1$ à $C_4$)-silyle, cyano, amino, alkyl (en $C_1$ à $C_4$)-amino, di-alkyl (en $C_1$ à $C_4$)-amino, acyl (en $C_1$ à $C_4$)-amino et(ou) les atomes d'halogène;
un groupe aroyle en $C_6$ à $C_{10}$ portant éventuellement un à trois substituants choisis parmi les radicaux alkyle en $C_1$ à $C_4$ et alcoxy en $C_1$ à $C_4$, les groupes trifluorométhyle, nitro, hydroxyle, acyle en $C_1$ à $C_4$, carboxyle, alcoxy (en $C_1$ à $C_5$)-carbonyle, N,N-di-alkyl (en $C_1$ à $C_4$)-amido, cyano et di-alkyl (en $C_1$ à $C_4$)-amino et(ou) les atomes d'halogène;
un groupe aroyle hétérocyclique penta- ou hexagonal avec un à trois hétéro-atomes, éventuellement benzo-conjugué, un groupe cycloalkyle en $C_3$ à $C_{12}$, acyle en $C_1$ à $C_8$ ou alcoxy (en $C_1$ à$C_5$)-carbonyle, un groupe aryle en $C_6$ à $C_{10}$ pouvant porter un à trois substituants choisis parmi les atomes d'halogène, les radicaux alkyle en $C_1$ à $C_4$ et alcoxy en $C_1$ à $C_4$ et les groupes hydroxyle, trifluorométhyle, acycle en $C_1$ à $C_4$, carboxyle, alcoxy (en $C_1$ à $C_5$)-carbonyle, cyano, nitro, amino, alkyl (en $C_1$ à $C_4$)-amino, di-alkyl (en $C_1$ à $C_4$)-amino, arylamino et acyl (en $C_1$ à $C_4$)-amino, ou un groupe aryle hétérocyclique penta- ou hexagonal, éventuellement benzo-conjugué, qui comprend un à trois atomes d'azote et le cas échéant un atome d'oxygène ou un atome de soufre, et
p vaut 2 ou 3;
d) un groupe de la formule IV

$$-NR^{10}R^{11} \qquad (IV),$$

dans laquelle $R^{10}$ et $R^{11}$ désignent chacun un atome d'hydrogène ou un groupe hydrocarboné en $C_1$ à $C_{12}$, pouvant être substitué par un ou deux groupes phényle, comportant éventuellement un à trois substituants choisis parmi les radicaux alkyle en $C_1$ à $C_4$ et alcoxy en $C_1$ à $C_4$, les groupes trifluorométhyle et(ou) nitro et(ou) les atomes d'halogène, un groupe naphtyle ou cycloalkyle en $C_3$ à $C_8$, comportant un à trois radicaux alkyle en $C_1$ à $C_4$, des groupes cycloalkyle en $C_5$ à $C_8$ pouvant être benzo-conjugués, ou un groupe bicycloalkyle en $C_7$ à $C_{10}$, portant un à trois radicaux alkyle en $C_1$ à $C_4$,
un groupe cycloalkyle en $C_3$ à $C_{12}$ portant éventuellement un à trois substituants choisis parmi les groupes hydrocarbonés en $C_1$ à $C_4$, pouvant comporter un groupe phényle mono- à tri-substitué par des atomes d'halogène et(ou) des groupes alkyle en $C_1$ à $C_4$, alcoxy en $C_1$ à $C_4$, trifluorométhyle, nitrile et nitro, ou un groupe cycloalkyle en $C_3$ à $C_6$, ou un groupe phényle mono- à tri-substitué par des groupes alkyle en $C_1$ à $C_4$, alcoxy en $C_1$ à $C_4$, trifluorométhyle, nitrile ou nitro et(ou) des atomes d'halogène ou par un groupe cycloalkyle en $C_3$ à $C_8$, un groupe alkyle bi- ou tri-cyclique en $C_7$ à $C_{10}$, pouvant être mono- à tri-substitué par des radicaux alkyle en $C_1$ à $C_4$ ou
un groupe cycloalkyle en $C_5$ à $C_7$ benzo-conjugué;
e) un groupe de la formule V

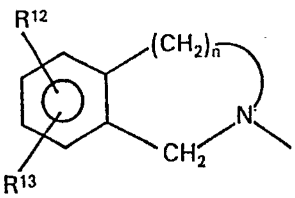
(V),

dans laquelle $R^{12}$ et $R^{13}$ désignent chacun un atome d'hydrogène ou d'halogène ou un radical alkyle en $C_1$ à $C_4$ ou alcoxy en $C_1$ à $C_4$ et n vaut 1, 2 ou 3;
f) un groupe de la formule VI

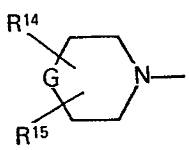
(VI),

dans laquelle G désigne un atome d'oxygène ou de soufre et $R^{14}$ et $R^{15}$ désignent chacun un atome d'hydrogène ou un radical alkyle en $C_1$ à $C_4$, avec les conditions que,
si $R^1$, $R^2$, A et B représentent des atomes d'hydrogène (et le groupe acyl-amino est en position para sur le noyau phénylique dans la formule I), $R^3$ n'est pas un groupe méthylène éventuellement alkyl (en $C_1$ à $C_5$)-substitué, et
si $R^2$, A et B désignent des atomes d'hydrogène, $R^1$ = H ou alkyle en $C_1$ à $C_3$ et $R^3$ désigne un pont méthylène éventuellement alkyl (en $C_1$ à $C_4$)-substitué, $R^4$ n'est pas un groupe de la formule IV, dans laquelle $R^{10}$ et $R^{11}$ représentent des atomes d'hydrogène ou des radicaux alkyle en $C_1$ à $C_4$ non substitués,
ainsi que des sels de ces dérivés et d'acides physiologiquement compatibles, caractérisé en ce qe:
a) on fait réagir un composé de la formule VII

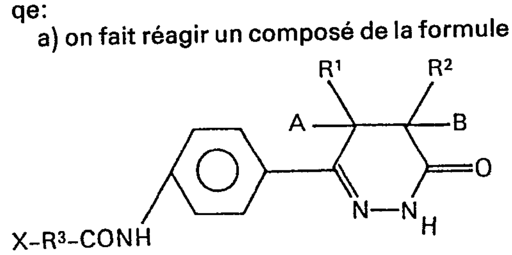
(VII),

$R^{11}$ est un atome d'hydrogène ou un radical alkyle en $C_1$ à $C_8$;

dans laquelle A, B, $R^1$, $R^2$ et $R^3$ possèdent les significations définies et X désigne un atome d'halogène, avec un composé de la formule VIII

$R^4$ - H (VIII),

dans laquelle $R^4$ possède les significations définies, ou

b) on soumet une aminophényl-pyridazinone de la formule IX

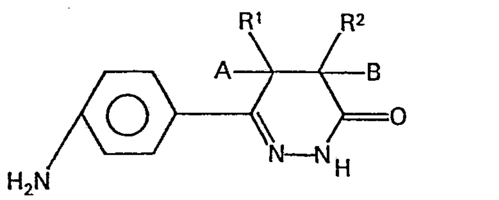

(IX),

dans laquelle A, B, $R^1$ et $R^2$ possèdent les significations définies, à une acylation connue en soi par un composé de la formule X

$R^4$ - $R^3$ - CO - Y·HY (X),

dans laquelle $R^3$ et $R^4$ possèdent les significations définies et Y désigne un atome de chlore ou de brome;

ou

c) on soumet un acide céto-carboxylique de la formule XI

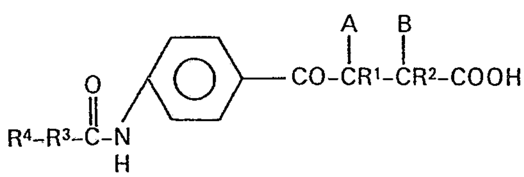

(XI),

dans laquelle A, B, $R^1$, $R^2$, $R^3$ et $R^4$ possèdent la signification définie, à une cyclisation à l'aide d'hydrazine;

d) lorsque A et B forment ensemble une liaison et $R^2$ désigne un radical méthyle, on fait réagir une di-hydro-pyridazinone de la formule I

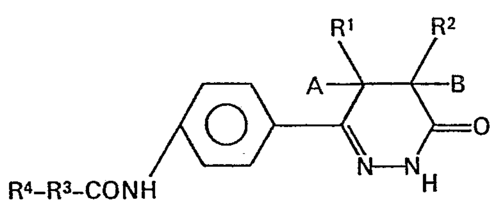

(I),

dans laquelle $R^1$, $R^3$ et $R^4$ possèdent les significations définies précédemment et A=B=$R^2$=H, avec l'aldéhyde formique,

les composés obtenus pouvant, le cas échéant, être transformés en leurs sels d'acides physiologiquement acceptables.

2. Procédé suivant la revendication 1, caractérisé en ce qu'il sert à préparer des dérivés de 6-(acylaminoaryl)-3(2H)-pyridazinones de la formule I selon la revendication 1, dans laquelle

A et B sont des atomes d'hydrogène ou forment ensemble une liaison,

$R^1$ est un atome d'hydrogène ou un radical méthyle et

$R^2$ est un atome d'hydrogène ou, lorsque A=B=H, $R^1$ et $R^2$ forment ensemble un groupe alkylène en $C_1$ ou $C_2$,

$R^3$ désigne un groupe alkylène à chaîne droite en $C_1$ à $C_4$, pouvant être substitué par un radical alkyle en $C_1$ à $C_3$ ou deux radicaux méthyle,

$R^4$ désigne

a) un groupe imidazole-1-yle;

b) un groupe de la formule II, dans laquelle (D et E possèdent la signification définie et)

$R^5$ désigne un atome d'hydrogène, un radical alkyle en $C_1$ à $C_4$, pouvant être substitué par un groupe phényle, un groupe cycloalkyle en $C_3$ à $C_6$, un groupe phényle pouvant être substitué par un ou deux radicaux alkyle en $C_1$ à $C_4$ ou alcoxy en $C_1$ à $C_4$ ou atomes d'halogène et(ou) par un groupe nitrile, nitro ou trifluorométhyle, ou un groupe de la formule $R^7R^8N$, dans laquelle $R^7$ est un atome d'hydrogène ou un groupe phényle pouvant être substitué par un ou deux atomes d'halogène ou radicaux alkyle en $C_1$ à $C_4$ ou alcoxy en $C_1$ à $C_4$ et(ou) par un groupe nitrile et(ou) nitro et $R^8$ est un groupe acyle en $C_1$ à $C_4$ ou benzoyle ou $R^7$ et $R^8$ forment avec l'atome d'azote adjacent un groupe benzimidazole-2-one-1-yle, et

$R^6$ est un atome d'hydrogène ou un groupe alkyle en $C_1$ à $C_4$, hydroxyle, acyle en $C_1$ à $C_4$, alcoxy (en $C_1$ à $C_5$)-carbonyle ou cyano ou

$R^5$ et $R^6$ forment ensemble un pont alkylène en $C_1$ à $C_4$, le composé bicyclique formé pouvant être substitué par un à trois radicaux méthyle,

m vaut 0, 1 ou 2;

c) un groupe de la fourmule III, dans laquelle

$R^9$ désigne un groupe hydrocarboné en $C_1$ à $C_3$, substitué par un groupe naphtyle ou phényle, le groupe phényle pouvant porter un ou deux atomes d'halogène, radicaux alkyle en $C_1$ à $C_4$ ou alcoxy en $C_1$ à $C_4$ ou un groupe trifluorométhyle, nitro, hydroxyle, acyle en $C_1$ à $C_4$, alcoxy (en $C_1$ à $C_5$)-carbonyle et(ou) cyano, un groupe naphtyle, gène (et le groupe acyl-amino est en position para sur le noyau phénylique dans la formule I), un groupe phényle mono- à tri-substitué par des atomes d'halogène ou radicaux alkyle en $C_1$ à $C_4$ ou alcoxy en $C_1$ à $C_4$ et(ou) mono-substitué par un groupe trifluorométhyle ou un groupe acyle en $C_1$ à $C_4$, ou un groupe aryle hexagonal hétérocyclique avec un ou deux atomes d'azote et

p vaut 2;

d) ($R^4$) représente un groupe de la formule IV, dans laquelle

$R^{10}$ désigne un radical alkyle en $C_1$ à $C_8$, éventuellement substitué par un grouipe phényle pouvant porter un ou deux radicaux alkyle en $C_1$ à $C_4$ et(ou) atomes d'halogène, un groupe cycloalkyle en $C_3$ à $C_{12}$ pouvant être substitué par un à trois radicaux méthyle ou un groupe phényle ou benzyle, un groupe alkyle bi- oiu tri-cyclique en $C_7$ à $C_{10}$ pouvant être substitué par un à trois radicaux méthyle ou un groupe cycloalkyle en $C_5$ à $C_7$ benzo-conjugué, et

e) ($R^4$) est un groupe de la formule V, dans laquelle $R^{12}$ et $R^{13}$ désignent chacun un atome d'hydrogène ou d'halogène ou un radical alkyle en $C_1$ à $C_4$ et n vaut 1 ou 2;

f) ($R^4$) est un groupe de la formule VI, dans laquelle G désigne un atome d'oxygène ou de soufre et $R^{14}$ et $R^{15}$ désignent chacun un atome d'hydrogène ou un radical méthyle,
avec les conditions que, lorsque $R^1=R^2=A=B=H$, $R^3$ n'est pas un groupe méthylène éventuellement substitué par un radical alkyle en $C_1$ à $C_3$, et lorsque $R^2=A=B=H$, $R^1=H$ ou $-CH^3$ est un groupe méthylène éventuellement substitué par un radical alkyle en $C_1$ à $C_3$, $R^4$ ne doit pas signifier un groupe de la formule IV, dans laquelle $R^{10}$ et $R^{11}$ sont des atomes d'hydrogène ou des radicaux alkyle en $C_1$ à $C_4$ non substitués, ainsi que des sels de ces dérivés et d'acides physiologiquement acceptables.